(19) Europäisches Patentamt
European Patent Office
Office européen des brevets

(11) Publication number: 0 391 814 A2

(12) # EUROPEAN PATENT APPLICATION

(21) Application number: 90420164.7

(22) Date of filing: 03.04.90

(51) Int. Cl.5: D01D 5/253, A61L 15/00, A61F 13/15

(30) Priority: 04.04.89 US 333651

(43) Date of publication of application: 10.10.90 Bulletin 90/41

(84) Designated Contracting States: GR

(71) Applicant: EASTMAN KODAK COMPANY
343 State Street
Rochester, New York 14650(US)

(72) Inventor: Phillips, Bobby Mal, c/o EASTMAN KODAK COMPANY
Patent Department, 343 State Street
Rochester, New York 14650(US)
Inventor: Bagrodia, Shriram, c/o Eastman Kodak Company
Patent dept., 343 State street,
Rochester New York 14650(US)
Inventor: Haile, William Alston, c/o Eastman Kodak Company
Patent Dept., 343 State Street,
Rochester, New York 14650(US)
Inventor: Hall, Harry Probert, c/o Eastman Kodak Company,
Patent Dept. 343 State Street,
Rochester, New York 14650(US)
Inventor: Casey, David Augustus, c/o Eastman Kodak Company,
Patent Dept. 343 State Street,
Rochester, New York 14650(US)

(74) Representative: Parent, Yves et al
Kodak-Pathé Département Brevets et Licences Centre de Recherches et de Technologie Zone Industrielle
F-71102 Chalon-sur-Saône Cédex(FR)

(54) **Fibers capable of spontaneously transporting fluids.**

(57) Disclosed are fibers that are capable of spontaneously transporting certain fluids, for example aqueous fluids, such as water, on their surfaces. The fibers, especially in the form of tow, can be incorporated into absorbant articles, such as diapers, in order to transport fluids to more effectively utilize the absorbant portion of the article.

EP 0 391 814 A2

Xerox Copy Centre

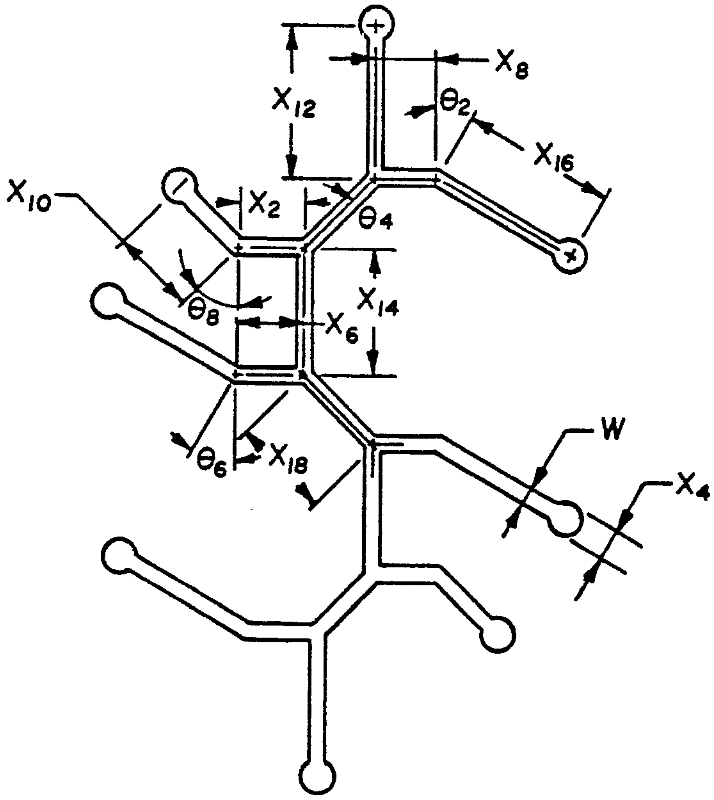


Fig. 3

# FIBERS CAPABLE OF SPONTANEOUSLY TRANSPORTING FLUIDS

## Field of the Invention

This invention concerns fibers that are capable of spontaneously transporting water on their surfaces and useful structures made from such fibers.

## Background of the Invention

Presently available absorbant articles such as diapers, sanitary napkins, incontinence briefs, and the like are generally very good at absorbing aqueous fluids such as urine and blood. However, during typical use such articles become saturated at the impingement zone while other zones removed from the impingement zone will remain dry. As a result, a substantial portion of the total absorbant capabilities of such articles remains unused. Thus, it would be highly desirable to have a means for transporting the aqueous fluids from the impingement zone to other areas of the absorbant article to more fully utilize the article's total absorbant capability. We have discovered such a means by the use of certain fibers that are capable of transporting aqueous fluids on their surfaces.

Liquid transport behavior phenomena in single fibers has been studied to a limited extent in the prior art (see, for example, A.M. Schwartz & F.W. Minor, J. Coll. Sci., 14, 572 (1959)).

There are several factors which influence the flow of liquids in fibrous structures. The geometry of the pore-structure in the fabrics (capillarity), the nature of the solid surface (surface free energy, contact angle), the geometry of the solid surface (surface roughness, grooves, etc.), the chemical/physical treatment of the solid surface (caustic hydrolysis, plasma treatment, grafting, application of hydrophobic/hydrophilic finishes), and the chemical nature of the fluid all can influence liquid transport phenomena in fibrous structures.

We have discovered fibers that have a unique combination of properties that allows for spontaneous transport of aqueous fluids such as water on their surfaces. Heretofore, fibers capable of spontaneously transporting aqueous fluids such as water have been unknown.

## Summary of the Invention

The present invention is directed to a synthetic fiber which is capable of spontaneously transporting water on the surface thereof.

The fiber of the present invention satisfies the following equation

$(1 - X \cos \theta_a) < 0$,

wherein

$\theta_a$ is the advancing contact angle of water measured on a flat film made from the same material as the fiber and having the same surface treatment, if any,

X is a shape factor of the fiber cross-section that satisfies the following equation

$$X = \frac{P_w}{4r + (\pi - 2)D}$$

wherein

$P_w$ is the wetted perimeter of the fiber and r is the radius of the circumscribed circle circumscribing the fiber cross-section and D is the minor axis dimension across the fiber cross-section.

It is preferred that $2\frac{r}{D}$ is greater than 1, it is more preferred that $2\frac{r}{D}$ is between 1.5 and 5.

It is also preferred that the fiber of the present invention satisfies the following equation :

$$\gamma_{LA} \cdot \frac{12\pi \cdot 10^{-4}}{\sqrt{\rho}} \cdot \sqrt{dpf} \cdot (1 - X \cos \theta_a) \leq -0.3,$$

wherein $\gamma_{LA}$ is the surface tension of water in air in dynes/cm, $\rho$ is the fiber density in grams/cc, and dpf is the denier of the single fiber.

It is preferred that X is greater than 1.2, preferably between about 1.2 and about 5, most preferably between about 1.5 and about 3.

## Brief Description of the Drawings

Figure 1A - illustration of the behavior of a drop of an aqueous fluid on a conventional fiber that is not spontaneously transportable after the ellipsoidal shape forms (t = 0). Angle $\theta$ illustrates a typical contact angle of a drop of liquid on a fiber. The arrows labelled "LFA" indicate the location of the liquid-fiber-air interface.

Figure 1B - illustration of the behavior of a drop of an aqueous fluid on a conventional fiber that is not spontaneously transportable at time = $t_1$ ($t_1$ >0). The angle $\theta$ remains the same as in Figure 1A. The arrows labelled "LFA" indicate the location of the liquid-fiber-air interface.

Figure 1C - illustration of the behavior of a drop of an aqueous fluid on a conventional fiber that is not spontaneously surface transportable at time = $t_2$ ($t_2$ >$t_1$ ). The angle $\theta$ remains the same as in Figure 1A. The arrows labelled "LFA" indicate the location of the liquid-fiber-air interface.

Figure 2A - illustration of the behavior of a drop of an aqueous fluid which has just contacted a fiber that is spontaneously transportable at time = 0. The arrows labelled "LFA" indicate the location of the liquid fiber-air interface.

Figure 2B - illustration of the behavior of a drop of an aqueous fluid on a fiber that is spontaneously transportable at time = $t_1$ ($t_1$ >0). The arrows labelled "LFA" indicate the location of the liquid-fiber-air interface.

Figure 2C - illustration of the behavior of a drop of an aqueous fluid on a fiber that is spontaneously transportable at time = $t_2$ ($t_2$ >$t_1$). The arrows labelled "LFA" indicate the location of the liquid-fiber-air interface.

Figure 3 - schematic representation of an orifice of a spinneret useful for producing a spontaneously transportable fiber.

Figure 4 - schematic representation of an orifice of a spinneret useful for producing a spontaneously transportable fiber.

Figure 5 - schematic representation of an orifice of a spinneret useful for producing a spontaneously transportable fiber.

Figure 6 - schematic representation of an orifice of a spinneret useful for producing a spontaneously transportable fiber.

Figure 6B - schematic representation of an orifice of a spinneret useful for producing a spontaneously transportable fiber.

Figure 7 - schematic representation of an orifice of a spinneret having 2 repeating units, joined end to end, of the orifice as shown in Figure 3.

Figure 8 - schematic representation of an orifice of a spinneret having 4 repeating units, joined end to end, of the orifice as shown in Figure 3.

Figure 9 photomicrograph of a poly(ethylene terephthalate) fiber cross-section made using a spinneret having an orifice as illustrated in Figure 3 (specific dimensions of spinneret orifice described in Example 1).

Figure 10 - photomicrograph of a polypropylene fiber cross-section made using a spinneret having an orifice as illustrated in Figure 3 (specific dimensions of spinneret orifice described in Example 2).

Figure 11 - photomicrograph of a nylon 66 fiber cross-section made using a spinneret having an orifice as illustrated in Figure 3 (specific dimensions of spinneret orifice described in Example 2).

Figure 12 - schematic representation of a poly(ethylene terephthalate) fiber cross-section made using a spinneret having an orifice as illustrated in Figure 4 (specific dimensions of spinneret orifice described in Example 8).

Figure 13 - photomicrograph of a poly(ethylene terephthalate) fiber cross-section made using a spinneret having an orifice as illustrated in Figure 5 (specific dimensions of spinneret orifice described in Example 9).

Figure 14 - photomicrograph of a poly(ethylene terephthalate) fiber cross-section made using a spinneret having an orifice as illustrated in Figure 7 (specific dimensions of spinneret orifice described in Example 10).

Figure 15 - photomicrograph of a poly(ethylene terephthalate) fiber cross-section made using a spinneret having an orifice as illustrated in Figure 8 (specific dimensions of spinneret orifice described in Example 11).

Figure 16 - schematic representation of a fiber cross-section made using a spinneret having an orifice as illustrated in Figure 3 (Example 1). Exemplified is a typical means of determining the shape factor X.

Figure 17 - photomicrograph of a poly(ethylene terephthalate) fiber cross-section made using a spinneret having an orifice as illustrated in Figure 6 (specific dimensions of spinneret orifice described in Example 12).

Figure 17B - schematic representation of a poly(ethylene terephthalate) fiber cross-section made using a spinneret having an orifice as illustrated in Figure 6B (specific dimensions of spinneret orifice described in Example 13).

Figure 18A - a schematic representation of the top view of a diaper.

Figure 18B - a schematic representation of an exploded side view of a diaper along section IB of the major axis of the diaper.

Figure 19 - a schematic representation of an exploded side view of a diaper along the major axis of the diaper. The tow made from fibers of the present invention is placed below the top sheet and above the absorbant core.

Figure 20 - a schematic representation of an exploded side view of a diaper along the major axis of the diaper. The tow made from fibers of the present invention is placed below the absorbant core and above the back sheet.

Figure 21 - a schematic representation of an exploded side view of a diaper along the major axis of the diaper. The tow made from fibers of the present invention is placed within the absorbant core.

Figure 22 - a schematic representation of an exploded side view of a diaper along the major axis of the diaper. The staple fibers made from fibers of the present invention is in the absorbant core.

Figure 23A - a schematic representation of the top view of a diaper. The lines in the cut-away view represent tow made from fibers of the present invention which are substantially parallel and running essentially the entire length of the diaper.

Figure 23B - a schematic representation of the top view of a diaper. The lines in the cut-away view represent tow made from fibers of the present invention which are substantially parallel and extending more than half the length of the diaper.

Figure 24 - a schematic representation of the top view of a diaper. The lines in the cut-away view represent tightly compacted tow (made from fibers of the present invention) in the impingement zone and the tow is flared at the end.

Figure 25 - a schematic representation of the top view of a diaper. The lines in the cut-away view represent tow made from fibers of the present invention. The major axis of the tow is inclined at an angle of 30° with respect to the major axis of the diaper.

Figure 26 - a schematic representation of an exploded side view of a diaper along the major axis of the diaper. The tow made from fibers of the present invention is placed above and below the absorbant core.

Figure 27 - graph of the ink holding capacity in grams (g) versus cartridge density in grams per cubic centimeter (g/cc) for an ink cartridge made from fibers of the present invention (line labeled "4SW") and for an ink cartridge made from fibers of the prior art of round cross-section (line labeled "round").

Figure 28 - graph of the percent ink remaining versus cartridge density (g/cc) for an ink cartridge made from fibers of the present invention (line labeled "4SW") and for an ink cartridge made from fibers of the prior art of round cross-section (line labeled "round").

Figure 29 - graph of the useable ink (g) versus cartridge density (g/cc) for an ink cartridge made from fibers of the present invention (line labeled "4SW") and for an ink cartridge made from fibers of the prior art of round cross-section (line labeled "round").

Figure 30 - graph of the ratio of useable ink (g)/fiber weight (g) versus cartridge density (g/cc) for an ink cartridge made from fibers of the present invention (line labeled "4SW") and for an ink cartridge made from fibers of the prior art of round cross-section (line labeled "round").

Figure 31A - a schematic representation of a desirable groove in a fiber cross-section.

Figure 31B - a schematic representation of a desirable groove in a fiber cross-section.

Figure 31C - a schematic representation of a desirable groove in a fiber cross-section illustrating the groove completely filled with fluid.

Figure 32A - a schematic representation of a groove where bridging is possible in the fiber cross-section.

Figure 32B - a schematic representation of a groove where bridging is possible in the fiber cross-section.

Figure 32C - a schematic representation of a groove illustrating bridging of the groove by a fluid.

Detailed Description of the Invention

The three important variables fundamental to the liquid transport behavior are (a) surface tension of the liquid, (b) wettability or the contact angle of the solid with the liquid, and (c) the geometry of the solid surface. Typically, the wettability of a solid surface by a liquid can be characterized by the contact angle that the liquid surface (gas-liquid interface) makes with the solid surface (gas-solid surface). Typically, a drop of liquid placed on a solid surface makes a contact angle, $\theta$, with the solid surface, as seen in Figure 1A. If this contact angle is less than 90°, then the solid is considered to be wet by the liquid. However, if the contact angle is greater than 90°, such as with water on Teflon surface, the solid is not wet by the liquid. Thus, it is desired to have a minimum contact angle for enhanced wetting, but definitely, it must be less than 90°. However, the contact angle also depends on surface inhomogeneities (chemical and physical, such as roughness), contamination, chemical/physical treatment of the solid surface, as well as the nature of the liquid surface and its contamination. Surface free energy of the solid also influences the wetting behavior. The lower the surface energy of the solid, the more difficult it is to wet the solid by liquids having high surface tension. Thus, for example, Teflon, which has low surface energy does not wet with water. (Contact angle for Teflon-water system is 112°.) However, it is possible to treat the surface of Teflon with a monomolecular film of protein, which significantly enhances the wetting behavior. Thus, it is possible to modify the surface energy of fiber surfaces by appropriate lubricants/finishes to enhance liquid transport. The contact angle of polyethylene terephthalate (PET), Nylon 66, and polypropylene with water is 80°, 71°, and 108°, respectively. Thus, Nylon 66 is more wettable than PET. However, for polypropylene, the contact angle is >90°, and thus is nonwettable with water.

The second property of fundamental importance to the phenomena of liquid transport is surface tension of the liquid.

The third property of fundamental importance to the phenomena of liquid transport is the geometry of the solid surface. Although it is known that grooves enhance fluid transport in general, we have discovered particular geometries and arrangements of deep and narrow grooves on fibers and treatments thereof which allow for the spontaneous surface transport of aqueous fluids in single fibers. Thus we have discovered fibers with a combination of properties wherein an individual fiber is capable of spontaneously transporting water on its surface.

The particular geometry of the deep and narrow grooves is very important. For example, as shown in Figures 31A, 31B and 31C, grooves which have the feature that the width of the groove at any depth is equal to or less than the width of the groove at the mouth of the groove are preferred over those grooves which do not meet this criterion (e.g., grooves as shown in Figures 32A, 32B and 32C). If the preferred groove is not achieved "bridging" of the liquid across the restriction is possible and thereby the effective wetted perimeter (Pw) is reduced. Accordingly, it is preferred that Pw is substantially equal to the geometric perimeter.

"Spontaneously transportable" and derivative terms thereof refer to the behavior of a fluid in general and in particular a drop of fluid, typically water, when it is brought into contact with a single fiber such that the drop spreads along the fiber. Such behavior is contrasted with the normal behavior of the drop which forms a static ellipsoidal shape with a unique contact angle at the intersection of the liquid and the solid fiber. It is obvious that the formation of the ellipsoidal drop takes a very short time but remains stationary thereafter. Figures 1A-1c and 2A-2C illustrate the fundamental difference in these two behaviors. The key factor is the movement of the location of the air, liquid, solid interface with time. If such interface moves just after contact of the liquid with the fiber, then the fiber is spontaneously transportable; if such interface is stationary, the fiber is not spontaneously transportable. The spontaneously transportable phenomenon is easily visible to the naked eye for large filaments (>20 denier per filament (dpf) or >22.22 dtex) but a microscope may be necessary to view the fibers if they are less than 22.22 dtex (20 dpf). Colored fluids are more easily seen but the spontaneously transportable phenomenon is not dependent on the color. It is possible to have sections of the circumference of the fiber on which the fluid moves faster than other sections. In such case the air, liquid, solid interface actually extends over a length of the fiber. Thus, such fibers are also spontaneously transportable in that the air, liquid, solid interface is moving as opposed to stationary.

Spontaneous transportability is basically a surface phenomenon; that is the movement of the fluid occurs on the surface of the fiber. However, it is possible and may in some cases be desirable to have the spontaneously transportable phenomenon occur in conjunction with absorption of the fluid into the fiber. The behavior visible to the naked eye will depend on the relative rate of absorption vs. spontaneous transportability. For example, if the relative rate of absorption is large such that most of the fluid is absorbed into the fiber, the liquid drop will disappear with very little movement of the air, liquid, solid interface along the fiber surface whereas if the rate of absorption is small compared to the rate of spontaneous transportability the observed behavior will be like that depicted in Figures 2A through 2C. In Figure 2A, a drop of aqueous fluid is just placed on the fiber (time = 0). In Figure 2B, a time interval has elapsed (time = $t_1$) and the fluid starts to be spontaneously transported. In Figure 2C, a second time interval has passed (time = $t_2$) and the fluid has been spontaneously transported along the fiber surface further than at time = $t_1$.

A fiber of the present invention is capable of spontaneously transporting water on the surface thereof. Distilled water can be employed to test the spontaneous transportability phenomenon; however, it is often desirable to incorporate a minor amount of a colorant into the water to better visualize the spontaneous transport of the water, so long as the water with colorant behaves substantially the same as pure water under test conditions. We have found aqueous Syltint Poly Red (trademark) from Milliken Chemicals to be a useful solution to test the spontaneous transportability phenomenon. The Syltint Poly Red solution can be used undiluted or diluted significantly, e.g., up to about 50x with water.

In addition to being capable of transporting water, a fiber of the present invention is also capable of spontaneously transporting a multitude of other aqueous fluids. Aqueous fluids are those fluids comprising about 50% or more water by weight, preferred is about 75% or more water by weight, most preferred is about 90% or more water by weight. Preferred aqueous fluids are body fluids, especially human body fluids. Such preferred fluids include, but are not limited to, blood, urine, perspiration, and the like. Other preferred aqueous fluids include, for example, aqueous inks.

In addition to being able to transport aqueous fluids, a fiber of the present invention is also capable of transporting an alcoholic fluid on its surface. Alcoholic fluids are those fluids comprising greater than about 50% by weight of an alcoholic compound of the formula R-OH

wherein R is an aliphatic or aromatic group containing up to 12 carbon atoms. It is preferred that R is an alkyl group of 1 to 6 carbon atoms, more preferred is 1 to 4 carbon atoms. Examples of alcohols include methanol, ethanol, n-propanol and iso-propanol. Preferred alcoholic fluids comprise about 70% or more by weight of a suitable alcohol. Preferred alcoholic fluids include antimicrobial agents, such as disinfectants, and alcohol-based inks.

Accordingly, the present invention is also directed to a process for spontaneously transporting an aqueous fluid (which includes water) or an alcoholic fluid on the surface thereof. Therefore, a process of the present invention can be described as a process for spontaneously transporting an aqueous fluid comprising contacting a fiber of the present invention with an aqueous fluid. Furthermore, another process of the present invention can be described as a process for spontaneously transporting an alcoholic fluid comprising contacting a fiber of the present invention with an alcoholic fluid. Once the aqueous fluid or alcoholic fluid contacts the fiber, said aqueous fluid or alcoholic fluid will be spontaneously transported. In many applications, it is preferred to have a portion of the fiber in contact with a source of the aqueous fluid and a different portion of the fiber in contact with a sink (the term "sink" will be defined hereinafter).

Fibers of this invention have the uniquely desirable feature of spontaneously transporting aqueous or alcoholic fluids on their surfaces. Since all of these fibers have finite length, e.g., a tow in a diaper which starts and stops at the ends of the diaper or a staple fiber of some specified cut length, the ability to move fluid ceases once the fluid reaches the ends of the fibers unless "sinks" for the fluid are provided. Sinks may be, for example, fluff pulp or superabsorbant gels, powders or fibers. Ideally, to maximize the utility of this invention, three key features are desired:

(1) a source of the appropriate fluid to be moved,

(2) the spontaneous surface transport of such fluids which initiates the movement of the fluid and fills the conduits through which the fluid moves after the fiber surface becomes "full" of fluid and the spontaneous driving forces no longer exist, and

(3) a sink or sinks for such fluid which are in intimate contact with the fiber at one or more locations along the length of each individual fiber.

For example, the practical significance of these three features can be seen in a diaper within the scope of the present invention during typical use. The source of the fluid is urine and is deposited in significant quantities in a reasonably periodic manner. After the first deposit the urine will be transported spontaneously along each fiber until such time as the source dries up (needs to be in contact at least about 10 seconds) or the fluid contacts a sink. As used herein the term "sink" can be defined as a structure which has a greater affinity for the aqueous fluid than the fiber. Assuming the source of fluid still exists the fiber will now act as a conduit to the sink until such time as the source dries up. It is clear that the locations of the sinks need to be removed from the location of the source if significant movement is desired (e.g. outer area of the diaper). Properly designed capillary structures of round cross-section filaments can exhibit spontaneous fluid movement However, the capillary structure depends on the location of the adjacent filaments and if they happen to move or be out of position no fluid movement takes place. A unique feature of the present invention is that the individual filaments spontaneous transport aqueous fluids without the need for adjacent filaments. This allows for many benefits such as for the movement of fluids over a much wider surface area. Usually more than one urination occurs before a diaper is changed. The second urination (source - at the impingement zone) will again be transported through the fiber conduits to appropriate sinks. The spontaneously transportable feature is probably of less significance the second time than the first urination because the conduits are partially or totally full of fluid. However, without the spontaneously transportable feature relatively little fluid movement takes place and the source section of the diaper (i.e., the impingement zone) remains very wet whereas the rest of the diaper remains very dry.

The fibers can the present invention can be comprised of any material known in the art capable of having a cross-section of the desired geometry. Preferred materials for use in the present invention are polyesters.

The preferred polyester materials useful in the present invention are polyesters or copolyesters that are well known in the art and can be prepared using standard techniques, such as, by polymerizing dicarboxylic acids or esters thereof and glycols. The dicarboxylic acid compounds used in the production of polyesters and copolyesters are well known to those skilled in the art and illustratively include terephthalic acid, isophthalic acid, p,p′-diphenyldicarboxylic acid, p,p′-dicarboxydiphenyl ethane, p,p′-dicarboxydiphenyl hexane, p,p′-dicarboxydiphenyl ether, p,p′-dicarboxyphenoxy ethane, and the like, and the dialkylesters thereof that contain from 1 to about 5 carbon atoms in the alkyl groups thereof.

Suitable aliphatic glycols for the production of polyesters and copolyesters are the acyclic and alicyclic aliphatic glycols having from 2 to 10 carbon atoms, especially those represented by the general formula $HO(CH_2)_pOH$, wherein p is an integer having a value of from 2 to about 10, such as ethylene glycol, trimethylene glycol, tetramethylene glycol, and pentamethylene glycol, decamethylene glycol, and the like.

Other known suitable aliphatic glycols include 1,4-cyclohexanedimethanol, 3-ethyl-1,5-pentanediol, 1,4-xylylene, glycol, 2,2,4,4-tetramethyl-1,3-cyclobutanediol, and the like. One can also have present a hydroxylcarboxyl compound such as 4, hydroxybenzoic acid, 4-hydroxyethoxybenzoic acid, or any of the other hydroxylcarboxyl compounds known as useful to those skilled in the art.

It is also known that mixtures of the above dicarboxylic acid compounds or mixtures of the aliphatic glycols can be used and that a minor amount of the dicarboxylic acid component, generally up to about 10 mole percent, can be replaced by other acids or modifiers such as adipic acid, sebacic acid, or the esters thereof, or with modifiers that impart improved dyeability to the polymers. In addition one can also include pigments, delusterants or optical brighteners by the known procedures and in the known amounts.

The most preferred polyester for use in the present invention is poly(ethylene terephthalate) (PET).

Other materials that can be used to make the fibers of the present invention include polyamides such as a nylon, e.g., nylon 66 or nylon 6; polypropylene; polyethylene; and cellulose esters such as cellulose triacetate or cellulose diacetate.

A single fiber of the present invention preferably has a denier of between about 3 and about 1,000 (about 3.33 and about 1111.11 dtex ), more preferred is between about 10 and about 70 (about 11.11 and about 77.78 dtex).

The fibers of the present invention preferably have a surface treatment applied thereto. Such surface treatment may or may not be critical to obtain the required spontaneous transportability property. The nature and criticality of such surface treatment for any given fiber can be determined by a skilled artisan through routine experimentation using techniques known in the art and/or disclosed herein. A preferred surface treatment is a coating of a hydrophilic lubricant on the surface of the fiber. Such coating is typically uniformly applied at about a level of at least 0.05 weight percent, with about 0.1 to about 2 weight percent being preferred. Preferred hydrophilic lubricants include a potassium lauryl phosphate based lubricant comprising about 70 weight percent poly(ethylene glycol) 600 monolaurate. Another surface treatment is to subject the fibers to oxygen plasma treatment, as taught in, for example, Plastics Finishing and Decoration, Chapter 4, Ed. Don Satas, Van Nostrand Reinhold Company (1986).

The novel spinnerets of the present invention must have a specific geometry in order to produce fibers that will spontaneously transport aqueous fluids.

In Figure 3, W is between 0.064 millimeters (mm) and 0.12 mm.

$$X_2 \text{ is } 4W\,{}^{+4W}_{-1W};\ X_4 \text{ is } 2W \pm 0.5W;\ X_6 \text{ is } 6W\,{}^{+4W}_{-2W};\ X_8 \text{ is } 6W\,{}^{+5W}_{-2W};\ X_{10} \text{ is } 7W\,{}^{+5W}_{-2W};\ X_{12} \text{ is } 9W\,{}^{+5W}_{-1W};\ X_{14} \text{ is } 10W\,{}^{+5W}_{-2W};\ X_{16} \text{ is } 11W\,{}^{+5W}_{-2W};\ X_{18} \text{ is } 6W\,{}^{+5W}_{-2W};$$

$\theta_2$ is 30° ± 30°; $\theta_4$ is 45° ± 45°; $\theta_6$ is 30° ± 30°; and $\theta_8$ is 45° ± 45°.

In Figure 4, W is between 0.064 mm and 0.12 mm;

$$X_{20} \text{ is } 17W\,{}^{+5W}_{-2W};\ X_{22} \text{ is } 3W \pm W;\ X_{24} \text{ is } 4W \pm 2W;\ X_{26} \text{ is } 60W\,{}^{+8W}_{-4W};\ X_{28} \text{ is } 17W\,{}^{+5W}_{-2W};\ X_{30} \text{ is } 2W \pm 0.5W;\ X_{32} \text{ is } 72W\,{}^{+10W}_{-5W};\ \text{and } \theta_{10} \text{ is } 45° \pm 15°.\ \text{In addition, each}$$

and $\theta_{10}$ is 45° ± 15°. In addition, each Leg B can vary in length from 0 to

$$\frac{X_{26}}{2};$$

and each Leg A can vary in length from 0 to

$$\tan\,(90-\theta_{10})\left[\frac{X_{26}}{2} - X_{24}\right].$$

In Figure 5, W is between 0.064 mm and 0.12 mm;

$$X_{34} \text{ is } 2W \pm 0.5W;\ X_{36} \text{ is } 58W\,{}^{+20W}_{-10W};\ X_{38} \text{ is } 24W\,{}^{+20W}_{-6W};\ \theta_{12} \text{ is } 20°\,{}^{+15°}_{-10°};\ \theta_{14} \text{ is } \frac{180°-2\theta_{12}}{n-1};$$

and n = number of legs per 180° = 2 to 6.

In Figure 6, W is between 0.064 mm and 0.12 mm; $X_{42}$ is 6W ${}^{+4W}_{-2W}$; $X_{44}$ is 11W ± 5W; $X_{46}$ is 11W ± 5W; $X_{48}$ is 24W ± 10W; $X_{50}$ is 38W ± 13W; $X_{52}$ is 3W ${}^{+3W}_{-1W}$;

U

$X_{54}$ is 6W ${}^{+6W}_{-2W}$;

$X_{56}$ is 11W ± 5W $X_{58}$ is 7W ± 5W; $X_{60}$ is 17W ± 7W; $X_{62}$ is 28W ± 11W; $X_{64}$ is 24W ± 10W; $X_{66}$ is 17W ± 7W; $X_{68}$ is 2W ± 0.5W; $\theta_{16}$ is 45° ${}^{+30°}_{-15°}$;

$\theta_{18}$ is 45° ± 15°; and $\theta_{20}$ is 45° ± 15°.

In Figure 6B W is between 0.064 mm and 0.12 mm,

$$X_{72} \text{ is } 8W {}^{+4W}_{-2W},\ X_{74} \text{ is } 8W {}^{+4W}_{-2W},$$

$X_{76}$ is 12W ± 4W, $X_{78}$ is 8W ± 4W, $X_{80}$ is 24W ± 12W, $X_{82}$ is 18W ± 6W, $X_{84}$ is 8W ${}^{+4W}_{-2W}$,

$X_{86}$ is 16W ± 6W, $X_{88}$ is 24W ± 12W, $X_{90}$ is 18W ± 6W, $X_{92}$ is 2W ± 0.5W, $\theta_{22}$ is 135° ± 30°, $\theta_{24}$ is 90° $\pm {}^{45°}_{30°}$,

$\theta_{26}$ is 45° ± 15°, $\theta_{28}$ is 45° ± 15°, $\theta_{30}$ is 45° ± 15°, $\theta_{32}$ is 45° ± 15°, $\theta_{34}$ is 45° ± 15°, $\theta_{36}$ is 45° ± 15°, and $\theta_{38}$ is 45° ± 15°.

In Figure 7, the depicted spinneret orifice contains two repeat units of the spinneret orifice depicted in Figure 3, therefore, the same dimensions for Figure 3 apply to Figure 7. Likewise, in Figure 8, the depicted spinneret orifice contains four repeat units of the spinneret orifice depicted in Figure 3, therefore, the same dimension for Figure 3 applies to Figure 8.

Figure 16 illustrates the method for determining the shape factor, X, of the fiber cross-section. In Figure 16, r = 37.5 mm, $P_w$ = 355.1 mm, D = 49.6 mm; thus, for the fiber cross-section of Figure 16:

$$X = \frac{355.1}{4 \times 37.5 + (\pi - 2)\ 49.6} = 1.72 .$$

The fibers of the present invention are preferably incorporated into an absorbant article in which it is desired to move or transport aqueous fluids. Such absorbant articles include, but are not limited to, diapers, incontinence pads, feminine hygiene articles such as tampons, ink cartridges, wipes, and the like. Figure 18A shows a schematic representation of the top view of a typical diaper and Figure 18B shows an exploded side view of a typical diaper along the major axis of the diaper.

The fibers of the present invention can be in the form of crimped or uncrimped tows or staple fibers comprising a plurality of the fibers of the present invention.

An absorbant article of the present invention comprises two or more fibers of the present invention wherein at least part of said fibers are located near the center of said absorbant article and at least part of same said fibers are located away from the center of said absorbant article; and wherein said fibers are capable of being in contact with an aqueous fluid for about at least 10 seconds near the center of said absorbant article; and wherein away from the center of said absorbant article one or more sinks are present in said absorbant article that are in contact with said fiber. As used in this context, "near the center" of the absorbant article means the geometric center and the area consisting of 50 area % of the total article immediately surrounding said geometric center; "away from the center" of the absorbant article means the remaining 50 area % that is not near the center of the article. Preferred sinks are fluff pulp, superabsorbant material, and combinations thereof. It is preferred that said sinks are in contact with a given fiber near the end of such fiber in the area away from the center of the article. As used in this context the term "near the end" of a fiber refers to an actual end of a fiber or the area consisting of the end 10% of the length of the fiber.

A preferred absorbant article of the present invention comprises a diaper or incontinent pad having a major axis and a minor axis and a length in excess of a width which comprises a top sheet, a back sheet, and an absorbant core comprising at least one absorbant layer wherein said article further comprises the tow of the present invention. The tow may be crimped or uncrimped.

The tow in said absorbant article can be located in several different positions with several different spatial orientations. For example, the tow can be uniformly spread across all or part of the width of the article and the fibers of the tow can be substantially parallel to the major axis of the article and extend from about 1/2 to substantially the length of the article (see Figure 23B).

Alternatively, the fibers of the tow can be substantially parallel to the major axis of the diaper and extend substantially the length of the diaper (see Figure 23A).

By use of a tow of the fibers of the invention in an absorbant article such as a diaper, urine can be transported to a larger surface area on the diaper. Thus, the amount of superabsorbant material required in the diaper can be reduced and the diaper surface will be drier.

By utilizing the fibers of the present invention in a diaper construction, it is preferred that at least one of the following benefits be realized.

(i) The effective surface area of the diaper utilized for urine/aqueous fluid movement will increase by 5% to 30%.

(ii) The amount of superabsorbant material utilized in the diaper will reduce by 2% to 25%.

(iii) The diaper will be thinner by about 2% to 15%.

(iv) The strikethrough (seconds)/rewet (grams) responses as measured by the strikethrough/rewet test described in U.S. Patent 4,324,247 are improved with the strikethrough being reduced from about 2 to about 50% and the rewet being reduced from about 2 to about 70% when compared to equivalent structures without the fibers (tow) of this invention being present. This results in the interface between the diaper and the wearer remaining drier.

The fibers of the tow can be located in the absorbant article at any place which will result in an overall beneficial effect. For example, the fibers can be located between the top sheet and the absorbant core, incorporated into the absorbant core, between the absorbant core and the back sheet, or multiple combinations of the above.

The top sheet of the absorbant article of the present invention can be made of any material known in the art for such use. Such materials include, but are not limited to, polypropylene, polyethylene, polyethylene terephthalate, cellulose or rayon; preferred is polypropylene. The top sheet is the sheet which is designed to be in contact with the body during typical end uses. Such a top sheet is alternatively referred to in the art as a "facing sheet," and is typically comprised of a web of short and/or long fibers.

The back sheet of the absorbant article of the present invention can be made of any material known in the art for such use. Such materials include, but are not limited to, polyethylene, a polyester, or polypropylene; preferred is polyethylene. The back sheet is typically impervious to body fluids such as urine.

The absorbant core of the absorbant article of the present invention preferably comprises fluff pulp and, optionally, superabsorbant powder. Fluff pulp is used extensively in the art. Fluff pulp is a batt formed of loosely compacted short cellulose fibers, such as wood pulp fibers, or cotton linters, or mixtures thereof, which are primarily held together by interfiber bonds usually requiring no added adhesive although thermoplastic binder(s) may also be used. This batt is a low density coherent web of loosely compacted fibers preferably comminuted wood pulp fibers. Examples of absorbant powder are polyacrylates, acrylic acid based polymers, saponified starch, and polyacrylonitrile graft copolymers.

Other preferred embodiments of the absorbant article of the present invention include wherein the fibers of the tow are tightly compacted in the impingement zone such that the fibers are substantially in contact with each other, and toward each end of the length of the article the fibers of the tow flare and are substantially not in contact with each other (see Figure 24). In addition, the tow can have from one half to ten turns of twist in the impingement zone. The terms "impingement zone", "impinging area", and like terms refer to that area or zone where body fluid first contacts or impinges the absorbant article during its intended use. The impingement zone may be near the center of the absorbant article, away from the center, or overlapping both areas.

It is also contemplated that the fibers of the present invention can be in the form of staple fiber which may or may not be crimped. When in the form of staple fiber, a preferred absorbant article of the present invention comprises a diaper or incontinent pad having a major axis and a minor axis and a length in excess of a width comprising a top sheet, a back sheet, and an absorbant core comprising at least one absorbant layer wherein said core comprises an intimate blend of the staple fiber of the present invention (see Figure 22).

Another preferred embodiment of the absorbant article of the present invention is wherein the article contains up to three tows of the invention and wherein the major axis of each tow lies between $\pm 30^\circ$ around the major axis of the article and wherein the tows lie either just beneath the top sheet or lie intimately mixed with the absorbant core or lie adjacent to the back sheet (see Figure 25).

Another preferred embodiment of the absorbant article of the present invention is a two piece diaper wherein one piece contains tow of the invention and receives the impinging fluid during the diaper's intended use and is reusable, and wherein the second piece is a fluid storage element and is replaceable.

The absorbant article of this invention can optionally contain a tissue or low density spacer layer which is adjacent to the top sheet between the top sheet and absorbant core. In such case the tow preferably lies between the absorbant core and said tissue or density spacer.

In still another preferred embodiment of the absorbant article of the present invention the fibers of the tow are in intimate contact with part of the absorbant core located away from the impingement zone.

Other absorbant articles contemplated by the present invention (which may or may not have a specific impingement zone) in which the fibers of the present invention can be beneficial include, but are not limited to, a sweat absorbing headband or wristband, a surgical sponge, a wound dressing, a sweat absorbing insole for footwear, a general purpose wiping article, a fabric softener strip for use in clothes dryers, a wound drain or a surgical drain, a towel, a geotextile, athletic clothing such as athletic socks and jogging suits, a cosmetic applicator, a furniture polish applicator, a pap smear sampler, a throat culture sampler, a blood-analyzer test element, household and industrial deodorizers, humidifier fabric, moist filter media, orthopaedic cast liner, wipes for medical applications (e.g., containing an alcoholic fluid for use on the surface of skin) and the like.

Ink cartridges are typically made with tows of cellulose ester fibers and polyester fibers. Important criteria for ink cartridges are (i) ink holding capacity and (ii) effective utilization of the ink reservoir. The art of making ink cartridges is described in U.S. Patents 4,104,781, 4,286,005, and 3,715,254. The use of fiber bundles made from fibers of the present invention in these ink cartridges offer significant advantages of increased ink holding capacity and/or effective utilization of the ink reservoir due to the nature of the fiber cross-sections and the spontaneous surface transportable nature of the single fibers of the present invention.

In the geotextile field, one of the important functions of the geotextile material is to transport rain water and other aqueous fluids from unwanted regions of the land to distant areas. It is believed that due to the spontaneous surface transportable nature of the fibers of the present invention, articles made from these fibers will enhance in transporting aqueous fluids from one region to another area in geotextile applications.

In active sports and outdoor activities, it is important that the human body remain relatively dry for comfort. Generally, human sweat or perspiration causes a feeling of being "wet". One of the important functions of garments and other articles worn next to skin is then to rapidly transport the "sweat" or "perspiration" from the skin to the garment or article worn next to the skin. Furthermore, it is important that such garments and articles should not absorb the bulk of this "sweat"; otherwise, it will take a long time to remove or dry the aqueous fluids from such garments and articles. For example, garments or such articles made of cotton or cellulosic fibers have a very high water absorption capacity (7-10%) and thus may not be highly desirable in such applications. However, garments or such articles worn next to skin made from fibers of the present invention and/or those in conjunction with blends of other fiber types may be very desirable. The spontaneous surface transportable nature of the fibers of the present invention can lead to rapidly removing the "sweat" or "perspiration" from the human body and thereby keeping the body relatively dry. Thus, a sweat absorbing headband or wristband, an insole for footwear, a towel, athletic socks, jogging suit, etc. made from fibers of the present invention can be highly desirable.

The fibers of the present invention can be prepared by techniques known in the art and/or disclosed herein using a novel spinneret of the present invention or other spinneret that will result in a fiber cross-section of the appropriate geometry and properties.

In general, a process of the present invention can be described as a process for preparing a fiber of the present invention which comprises heating a material capable of forming a fiber at or above its melting point followed by extruding said heated material through at least one spinneret having at least one orifice capable of forming the desired fiber. The fiber may be drafted and/or thermally stabilized. The fiber thus formed may then optionally be treated with a surface treatment such as a hydrophilic coating or plasma treatment as described hereinbefore.

The absorbant articles of the present invention can be made by use of techniques known in the art, for example in U.S. Patents 4,573,986; 3,938,522; 4,102,340; 4,044,768; 4,282,874; 4,285,342; 4,333,463; 4,731,066; 4,681,577; 4,685,914; and 4,654,040; and/or by techniques disclosed herein. The tow of the present invention can be incorporated into the absorbant article at any location which will improve fluid movement so as to better utilize the absorbant materials of the article.

Spunbonded structures, well known in the art, can also be made from filament strands of the present invention. Care must be exercised in the calendaring step so as not to damage the cross-section of the fibers and thereby inhibit the spontaneous surface transport.

Continuous filament yarns of typical textile deniers and filament counts can also be made using the present invention. The yarns are useful in providing scrim fabrics which will spontaneously surface transport aqueous fluids.

The following examples are to illustrate the invention but should not be interpreted as a limitation thereon.

## EXAMPLES

### Example 1

Poly(ethylene terephthalate) (PET) polymer of 0.6 I.V. was used in this example. I.V. is the inherent viscosity as measured at 25 °C at a polymer concentration of 0.50 g/100 milliliters (mL) in a suitable solvent such as a mixture of 60% phenol and 40% tetrachloroethane by weight. The polymer was dried to a moisture level of ≤0.003 weight percent in a Patterson Conaform dryer at 120 °C for a period of 8 hours. The polymer was extruded at 283 °C through an Egan extruder, 1.5-inch (38.1 mm) diameter, with a length to-diameter ratio of 28:1. The fiber was extruded through an eight orifice spinneret wherein each orifice is as shown in Figure 3 wherein W is 0.084 mm, $X_2$ is 4W, $X_4$ is 2W, $X_6$ is 6W, $X_8$ is 6W, $X_{10}$ is 7W, $X_{12}$ is 9W, $X_{14}$ is 10W, $X_{16}$ is 11W, $X_{18}$ is 6W, $\theta_2$ is 0°, $\theta_4$ is 45°, $\theta_6$ is 30°, and $\theta_8$ is 45°. The polymer throughput was about 7 pounds (1b)/hour (3.18 kg per hour). The air quench system has a cross-flow configuration. The quench air velocity at the top of the screen was an average of 294 feet (ft)/minute (89.61 m/minute). At a distance of about 7 inches (177.8 mm) from the top of the screen the average velocity of the quench air was about 285 ft/minute (86.87 m/minute), and at a distance of about 14 inches (355.6 mm) from the top of the screen the average quench air velocity was about 279 ft/minute (85.04 m/minute). At about 21 inches (533.4 mm) from the top of the air screen the average air velocity was about 340 ft/minute (103.63 m/minute). The rest of the screen was blocked. Spinning lubricant was applied via ceramic kiss rolls. The lubricant has a general composition as follows: it is a potassium lauryl phosphate (PLP) based lubricant having poly(ethylene glycol) 600 monolaurate (70% by weight) and polyoxyethylene (5) potassium lauryl phosphate (30% by weight). An emulsion of the above lubricant with water (90%) was used as the spinning lubricant. The lubricant level on the fiber samples was about 1.5%. Fibers of 20 dpf (denier per filament) (22.22 dtex) were wound at 3,000 meters per minute (MPM) on a Barmag SW4SL winder. A photomicrograph of a cross section of this fiber is shown in Figure 9 (150x magnification). The single fiber was tested for spontaneous surface transportation of an aqueous solution which was aqueous Syltint Poly Red (obtained from Milliken Chemicals) which is 80 weight % water and 20 weight % red colorant. The single fiber of 20 dpf (22.22 dtex) spontaneously surface transported the above aqueous solution. The following denier per filament PET fibers were also made at different speeds as shown in Table 1 below:

Table 1

| dpf | Spin Speed (MPM) | Winder |
|---|---|---|
| 20 | 3,000 | Barmag |
| 40 | 1,500 | Leesona |
| 60 | 1,000 | Leesona |
| 120 | 500 | Leesona |
| 240 | 225 | Leesona |
| 400 | 150 | Leesona |

All the single fibers of above PET fiber with the dpf of 20, 40, 60, 120, 240, and 400 spontaneously surface transported the aqueous solution of Syltint Poly Red liquid. The value of the "X" parameter (as defined hereinbefore) for these fibers was about 1.7. PET film of 0.02 inch (0.508 mm) thickness was compression molded from the same polymer as that used for making the above fiber. Contact angle of distilled water on the above film was measured in air with a contact angle goniometer. The contact angle was 71.7°. Another sample of the same film as above was sprayed with the same lubricant as used for making the fiber in this example at about 1.5% level. The contact angle of distilled water on the PET film sprayed with the lubricant was about 7°. Thus, the factor $(1\text{-X} \cos \theta)$ in this case is $(1\text{-}1.7(\cos 7°)) = -0.69$, which is less than zero.

Example 2

Polyhexamethylene adipamide (nylon 66) was obtained from Du Pont [Zytel 42 (trademark)]. The polymer was extruded at 279 °C. A spinneret as shown in Figure 3 was used to form 46 dpf fiber at 255 meters/minute speed. The specific dimensions of the spinneret orifices were the same as described in Example 1 except that $\theta_2$ was 30° instead of 0°. The quenching conditions were the same as those for obtaining PET fiber as in Example 1. A photomicrograph of the fiber cross-section is shown in Figure 11 (150x magnification). The lubricant level on the fiber was about 1.8% by weight. The same lubricant as used in the PET fiber was used (Example 1). This Nylon 66 fiber spontaneously transported the aqueous Syltint Poly Red solution on the fiber surface. The value of the "X" parameter for this fiber was about 1.9. Nylon 66 film of 0.02 inch (0.508 mm) thickness was compression molded from the same polymer as that used for making the fiber of Example 2. Contact angle of distilled water on the above film was measured in air with a contact angle goniometer. The contact angle was 64°. Another sample of the same film as above was sprayed with the same lubricant as used for making the fiber in this example at about the 1.8% level. The contact angle of distilled water on the nylon 66 film sprayed with the lubricant was about 2°. Thus, the factor (1-X cos $\theta$) in this case is (1-1.9(cos 2°)) = -0.9, which is less than zero.

Example 3

Polypropylene polymer was obtained from Shell Company (Grade 5C14). It was extruded at 279°C. A spinneret as shown in Figure 3 was used to form 51 dpf (56.67 dtex) fiber at 2,000 MPM speed. The specific dimensions of the spinneret orifices were the same as in Example 2. The quenching conditions were the same as those for obtaining PET fiber. A photomicrograph of the fiber cross-section is shown in Figure 10 (375x magnification). The lubricant level on the fiber was 2.6%. The same lubricant as used in PET fiber was used (Example 1). The polypropylene fiber spontaneously transported the aqueous Syltint Poly Red solution on the fiber surface. This spontaneously transportable phenomenon along the fiber surface was also observed for a 10 dpf (11.11 dtex), single polypropylene fiber. The value of the "X" parameter for this fiber was about 2.2. Polypropylene film of 0.02 inch (0.508 mm) thickness was compression molded from the same polymer as that used for making the above fiber of Example 3. Contact angle of distilled water on the above film was measured in air with a contact angle goniometer. The contact angle was about 110°. Another sample of the same film as above was sprayed with the same lubricant as used for making the fiber in this example at about the 2.6% level. The contact angle of distilled water on the polypropylene film sprayed with the lubricant was 12°. Thus, the factor (1-X cos $\theta$) in this case is -1.1, which is less than zero.

Example 4

Cellulose acetate (Eastman Grade CA 398-30, Class I) was blended with PEG 400 polymer and small quantities of antioxidant and thermal stabilizer. The blend was melt extruded at 270°C. A spinneret as shown in Figure 3 was used to form 115 dpf (127.78 dtex) fiber at 540 meters/minute speed. The specific dimensions of the spinneret orifices were the same as in Example 2. No forced quench air was used. The lubricant level on the fiber was 1.6%. The same lubricant as used in the PET fibers (Example 1) was used. The cellulose acetate fiber spontaneously transported the aqueous Syltint Poly Red solution on the fiber surface. The value of the "X" parameter for this fiber was about 1.8.

Example 5 (Comparative)

PET fiber of Example 1 was made without any spinning lubricant at 20 dpf (22.22 dtex). A single fiber did not spontaneously transport the aqueous Syltint Poly Red solution along the fiber surface.

Example 6 (Comparative)

PET fiber of circular cross-section was made. The denier per filament of the fiber was 20 (22.22 dtex). It had about 1.5% of the lubricant used in Example 1. A single fiber did not spontaneously transport the aqueous Syltint Poly Red solution along the fiber surface.

Example 7

Poly(ethylene terephthalate) (PET) fiber of Example 5 (without any spinning lubricant) was treated with oxygen plasma for 30 seconds. Model "Plasmod" oxygen plasma equipment was used. Exciter power is provided by the RF generator operating at 13.56 MHz frequency. The plasma treatment was conducted at a constant level of 50 watts power. The oxygen plasma treated fiber spontaneously transported the aqueous Syltint Poly Red solution along the fiber. This fiber was tested again after washing five times and after 3 days and the spontaneously transportable behavior with the above aqueous solution was still observed. In order to determine the reduction in contact angle after the plasma treatment, a PET film of the same material as that of the fiber was subjected to the oxygen plasma treatment under the same conditions as those used for the fiber sample. The average contact angle of the oxygen plasma treated film with distilled water in air was observed to be 26° as measured by a contact angle goniometer. The corresponding contact angle for the control PET film (not exposed to the oxygen plasma) was 70°. The significant reduction in contact angle upon subjecting the untreated PET fiber to the oxygen plasma treatment renders it to be spontaneously surface transportable for aqueous solutions.

Example 8

Poly(ethylene terephthalate) (PET) polymer of 0.6 IV was used in this example. It was extruded through a spinneret having eight orifices as shown in Figure 4 wherein W is 0.084 mm, $X_{20}$ is 17W, $X_{22}$ is 3W, $X_{24}$ is 4W, $X_{26}$ is 60W, $X_{28}$ is 17W, $X_{30}$ is 2W, $X_{32}$ is 72W, $\theta_{10}$ is 45°, Leg B is 30W, and Leg A is 26W. The rest of the processing conditions were the same as those described in Example 1. A 100 dpf fiber (111.11 dtex) was spun at 600 MPM. A sketch of the cross-section of the fiber is shown in Figure 12. The lubricant level on the fiber was about 1%. The same lubricant as used in Example 1 was used. The above fiber spontaneously transported the aqueous Syltint Poly Red solution along the fiber surface. The value of the "X" parameter for this fiber was 1.5.

Example 9

Poly(ethylene terephthalate) polymer of 0.6 IV was used in this example. It was extruded through a spinneret having eight orifices as shown in Figure 5 wherein W is 0.10 mm, $X_{34}$ is 2W, $X_{36}$ is 58W, $X_{38}$ is 24W, $\theta_{12}$ is 20°, $\theta_{14}$ is 28°, and n is 6. The rest of the extruding and spinning conditions were the same as those described in Example 1. A photomicrograph of the fiber cross-section is shown in Figure 13 (585x magnification). A 20 dpf fiber (22.22 dtex) was spun at 3000 MPM. The lubricant level on the fiber was about 1.7%. The same lubricant as used in Example 1 was used. The above fiber spontaneously transported the aqueous Syltint Poly Red solution along the fiber surface. The value of the "X" parameter for this fiber was about 2.4.

Example 10

Poly(ethylene terephthalate) (PET) polymer of about 0.6 IV was used in this example. The polymer was extruded through a spinneret having four orifices as shown in Figure 7 wherein the dimensions of the orifices are repeats of the dimensions described in Example 2. The rest of the processing conditions were the same as those described in Example 1 unless otherwise stated. A 200 dpf (222.22 dtex) fiber was spun at 600 MPM. The polymer throughput was about 7 lbs/hr (3.18 kg/hr). An optical photomicrograph of the fiber is shown in Figure 14 (150x magnification). The lubricant level on the fiber was 2.0%. The same lubricant as used in Example 1 was used. The above fiber spontaneously transported the aqueous Syltint Poly Red solution along the fiber surface. The value of the "X" parameter for this fiber was about 2.2.

Example 11

Poly(ethylene terephthalate) (PET) polymer of 0.6 IV was used in this example. The polymer was extruded through a spinneret having two orifices as shown in Figure 8 wherein the dimensions of the orifices are repeats of the dimensions described in Example 2. The rest of the processing conditions were the same as those described in Example 1. A 364 dpf (404.44 dtex) fiber was spun at 600 MPM. The cross-section of the fiber is shown in Figure 15 (150x magnification). The lubricant level on the fiber was about 2.7%. The same lubricant as used in Example 1 was used. The above fiber spontaneously transported the aqueous Syltint Poly Red solution along the fiber surface. The value of the "X" parameter for this fiber was 2.1.

Example 12

Poly(ethylene terephthalate) (PET) polymer of 0.6 IV was used in this example. It was extruded through a spinneret having eight orifices as shown in Figure 6 wherein W is 0.10 mm, $X_{42}$ is 6W, $X_{44}$ is 11W, $X_{46}$ is 11W, $X_{48}$ is 24W, $X_{50}$ is 38W, $X_{52}$ is 3W, $X_{54}$ is 6W, $X_{56}$ is 11W, $X_{58}$ is 7W, $X_{60}$ is 17W, $X_{62}$ is 28W, $X_{64}$ is 24W, $X_{66}$ is 17W, $X_{68}$ is 2W, $\theta_{16}$ is 45°, $\theta_{18}$ is 45°, and $\theta_{20}$ is 45°. The rest of the processing conditions were the same as those described in Example 1. A 100 dpf (111.11 dtex) fiber was spun at 600 MPM. The cross-section of the fiber is shown in Figure 17. The lubricant level on the fiber was about 1%. The same lubricant as used in Example 1 was used. The above fiber spontaneously transported the aqueous Syltint Poly Red solution along the fiber surface. The value of the "X" parameter for this fiber was 1.3.

Example 13

PET polymer of 0.6 I.V. is used in this example. It is extruded through a spinneret having 8 orifices as shown in Figure 6B wherein W is 0.10 mm, $X_{72}$ is 8W, $X_{74}$ is 8W, $X_{76}$ is 12W, $X_{78}$ is 8W, $X_{80}$ is 24W, $X_{82}$ is 18W, $X_{84}$ is 8W, $X_{86}$ is 16W, $X_{88}$ is 24W, $X_{90}$ is 18W, $X_{92}$ is 2W, $\theta_{22}$ is 135°, $\theta_{24}$ is 90°, $\theta_{26}$ is 45°, $\theta_{28}$ is 45°, $\theta_{30}$ is 45°, $\theta_{32}$ is 45°, $\theta_{34}$ is 45°, $\theta_{36}$ is 45° and $\theta_{38}$ is 45°. A 20 dpf (22.22 dtex) fiber is spun at 3,000 m/min. The rest of the processing conditions are the same as those used in Example 1. The lubricant level on the fiber is about 1%. The cross-section of the fiber is shown in Figure 17B. This fiber spontaneously transports the aqueous Syltint Poly Red solution along the fiber surface. The "X" value for this fiber is about 2.1.

Example 14

A disposable absorbant article is prepared comprising (a) a liquid impervious backing sheet made of polyethylene, (b) a relatively hydrophobic, liquid pervious top sheet made of polypropylene, (c) a layered absorbant core positioned between said backing sheet and said top sheet, and (d) tow or fibers of the present invention. The cover or facing provided on the absorbant structure is a non-woven fabric having a high degree of moisture permeability. For example, the fabric may be polyester, polyethylene, polypropylene, nylon, rayon, or the like. Preferably, the fabric used for the cover is a lightweight fabric in the range of 0.3 to 5.0 oz./square yard (10.17 to 169.5 g/m$^2$) and with a density less than 0.3 g/cc. The most suitable fabrics have unusually high elongation, softness, and drape characteristics. Though the cover is moisture permeable, it is preferably of the type which after permeation of the moisture prevents strike-back of the body fluid when the absorbant structure is approaching saturation. The body of the cellulosic fibrous batt (fluff pulp) is substantially more wettable than the cover and tends to draw liquid away from the facing layer. The cellulosic batt may be wrapped in a tissue. It may not be necessary to have a tissue wrapping the cellulosic batt, but if the cellulosic batt is quite thick, such as an inch or more, it may be desirable to provide a tissue wrap to assist with maintenance of the desired shape of the absorbant structure. The cellulosic batt also contains a water-swellable, water-insoluble absorbant composition. The superabsorbant particles are generally in the form of a dry solid water-swellable, water-insoluble absorbant composition such an ionic complex of a water-soluble anionic polyelectrolyte and a polyvalent metal cation. Typical superabsorbant compositions are illustrated in U.S. Patent 4,090,013 to S.H. Ganslaw et al. and U.S. Patent 4,043,952 to S.H. Ganslaw et al. The superabsorbant material may be in the form of individual particles or strips of film to which the superabsorbant material is adhered to other known superabsorbant compositions. The superabsorbant material may be affixed to the base of the superabsorbant reservoir or may simply lie independently within the reservoir. The fibers of the present invention may be placed in a tow form or fiber bundle immediately below the top sheet as shown in Figure 19. By using the tow of the fibers of the present invention, the body fluid (e.g., urine) will be spread farther along the absorbant article (thus improving the strikethrough and rewet properties), thereby more effectively utilizing the available absorbant area and superabsorbant material and resulting in a drier skin-absorbant article interface.

Example 15

The components of the disposable absorbant article are the same as in Example 14. However, in this case the fibers of the present invention are placed in a tow form within the cellulosic batt (absorbant core) as shown in Figure 21. By using the tow of the fibers of the present invention, the body fluid (e.g., urine) will be spread farther along the absorbant article (thus improving the strikethrough and rewet properties), thereby more effectively utilizing the available absorbant area and superabsorbant material and resulting in a drier skin absorbant article interface.

Example 16

The components of the disposable absorbant article are the same as in Example 14. However, in this case the fibers of the present invention are placed in the tow form immediately below the cellulosic batt (absorbant core) as shown in Figure 20. By using the tow of the fibers of the present invention, the body fluid (e.g., urine) will be spread farther along the absorbant article (thus improving the strikethrough and rewet properties), thereby more effectively utilizing the available absorbant area and superabsorbant material and resulting in a drier skin-absorbant article interface.

Example 17

The components of the disposable absorbant article are the same as in Example 14. However, in this case the fibers of the present invention are placed in the layer containing the cellulosic batt (absorbant core). There is an intimate blend of the staple fiber of the present invention and fluff pulp (hydrophilic cellulosic fiber). The fibers of the present invention are in cut, staple form 0.25 inch (6.35 mm) to 6 inches (152.4 mm) in length (see Figure 22). By using the tow of the fibers of the present invention, the body fluid (e.g., urine) will be spread farther along the absorbant article (thus improving the strikethrough and rewet properties), thereby more effectively utilizing the available absorbant area and superabsorbant material and resulting in a drier skin absorbant article interface.

Example 18

The components of the disposable absorbant article are the same as in Example 14. However, in this case the fibers of the present invention are placed in the tow form above and below the cellulosic batt (absorbant core) as shown in Figure 26. By using the tow of the fibers of the present invention, the body fluid (e.g., urine) will be spread farther along the absorbant article (thus improving the strikethrough and rewet properties), thereby more effectively utilizing the available absorbant area and superabsorbant material and resulting in a drier skin-absorbant article interface.

Example 19

The components of the disposable absorbant article are the same as in Example 14. However, in this case the fibers of the present invention are in the tow form and tightly compacted in the impingement zone (the tow may also be twisted in the impingement zone) such that the fibers are substantially in contact with each other (thereby promoting rapid movement of urine or other bodily fluids along the fiber axis due to the combined action of the spontaneous surface transportable nature of these single fibers and the capillary flow in the void space between the fibers), and toward each end of the length of the article the fibers of the tow flare and are substantially not in contact with each other. One possible arrangement is shown in Figure 24. This arrangement will allow rapid movement of urine from the impingement zone to the outer areas of the diaper. By using the tow of the fibers of the present invention, the body fluid (e.g., urine) will be spread farther along the absorbant article (thus improving the strikethrough and rewet properties), thereby more effectively utilizing the available absorbant area and superabsorbant material and resulting in a drier skin-absorbant article interface.

Example 20

Tows of this invention are very useful for making ink reservoir cartridges for writing instruments which utilize aqueous based inks. 96/8 d/f PET yarns were made using the conditions of Example 1 except the lubricant level was 3.4%. These yarns were plied, drafted 1.5X, thermally stabilized, crimped and pulled into cylindrical cartridges (0.70 cm in diameter) such that the density in the cartridges ranged from about 0.10 g/cc to about 0.25 g/cc. Appropriate round cross-section PET control was made at 8 dpf (8.89 dtex) with 1% of the lubricant used in Example 1, crimped and pulled into the same size cylindrical cartridges such that the densities ranged from about 0.10 g/cc to about 0.25 g/cc. These cylindrical cartridges were cut to lengths of 7.95 cm and all of the testing was done using Sheaffer Skript (trademark) writing fluid, washable black #632.

Figure 27 shows the ink holding capacity versus cartridge density for cartridges made from fibers of the present invention and round fiber controls. This test basically involves dripping ink into the cartridges of known weight held in a vertical position and determining the amount of ink the cartridge holds when it begins to drip from the bottom of the cartridge. This weight in grams is called the ink holding capacity of the cartridge being tested. The improvement ranges from about 13% to 26% over the range of densities tested.

Figure 28 shows the percent ink remaining versus cartridge density for cartridges made from PET fibers of the present invention and round PET fiber controls. Percent ink remaining is defined as

$$\frac{\text{ink remaining in the cartridge after dewicking (g)}}{\text{ink holding capacity (g)}} \times 100$$

where the ink remaining in the cartridge after dewicking is determined by weighing the cartridge, filling it with ink (ink holding capacity), weighing the cartridge plus ink, contacting the bottom of the cartridge with Type F2 Buckeye Filter paper and dewicking until such time as no ink leaves the cartridge, weighing the cartridge plus ink remaining and finally subtracting the weight of the cartridge to determine the weight in grams of the ink remaining in the cartridge. Notice the clearly superior behavior of the cartridge containing fibers of the present invention.

Figure 29 shows useable ink versus cartridge density for cartridges made from fibers of the present invention and round fiber controls. This test involves dripping ink into the cartridge of known weight such that its ink holding capacity is equaled, contacting the bottom of the cartridge with Type F2 Buckeye Filter paper and dewicking until such time as no ink leaves the cartridge, weighing the cartridge plus unavailable ink and subtracting the weight of the unavailable ink (g) from the ink holding capacity (g) to determine the useable ink in grams. The improvement ranges from about 15% to about 30% over the range of densities tested.

Figure 30 shows the ratio of useable ink to fiber weight versus cartridge density for cartridges made from fibers of the present invention and round fiber controls. Notice the significant improvement of the cartridges made from fibers of the present invention.

The invention has been described in detail with particular reference to preferred embodiments thereof, but it will be understood that variations and modifications can be effected within the spirit and scope of the invention.

## Claims

1. A synthetic fiber which is capable of spontaneously transporting water on the surface thereof wherein said fiber satisfies the equation

$(1 - X \cos \theta_a) < 0$,

wherein

$\theta_a$ is the advancing contact angle of water measured on a flat film made from the same material as the fiber and having the same surface treatment, if any,

X is a shape factor of the fiber cross-section that satisfies the following equation

$$X = \frac{P_w}{4r + (\pi - 2)D}$$

wherein

$P_w$ is the wetted perimeter of the fiber and r is the radius of the circumscribed circle circumscribing the fiber cross-section and D is the minor axis dimension across the fiber cross-section.

2. The fiber of Claim 1 wherein $2\frac{r}{D}$ is between 1.5 and 5.

3. The fiber of Claim 1 which satisfies the equation

$$\gamma_{LA} \cdot \frac{12\pi \cdot 10^{-4}}{\sqrt{\rho}} \cdot \sqrt{dpf} \cdot (1 - X \cos \theta_a) \leq -0.3,$$

wherein $\gamma_{LA}$ is the surface tension of water in air in dynes/cm, $\rho$ is the fiber density in grams/cc, and dpf is the denier of the single fiber.

4. The fiber of Claim 1 wherein X is from about 1.2 to about 5.

5. The fiber of Claim 1 having a single fiber denier of between 10 and 70.

6. The fiber of Claim 1 comprised of a material selected from the group consisting of a polyester, polypropylene, polyethylene, a cellulose ester, and a nylon.

7. The fiber of Claim 6 having coated thereon a layer of a hydrophilic lubricant.

8. The fiber of Claim 1 wherein the width of each groove in the fiber cross-section at any depth in the groove is equal to or less than the width of the groove at its mouth.

9. An absorbant article comprising two or more fibers of Claim 1 wherein at least part of said fibers are located near the center of said absorbant article and at least part of same said fibers are located away from the center of said absorbant article; and wherein said fibers are capable of being in contact with an aqueous fluid for at least 10 seconds near the center of said absorbant article; and wherein away from the center of said absorbant article one or more sinks are present in said absorbant article that are in contact with said fibers.

10. A tow comprising a plurality of the fibers of Claim 1.

11. An absorbant article comprising a diaper or incontinent pad having a major axis and a minor axis and a length in excess of a width which comprises a top sheet, a back sheet, and an absorbant core comprising at least one absorbant layer wherein said article further comprises the tow of Claim 10.

12. The article of Claim 11 wherein said tow is uniformly spread across all or part of the width of the article and the fibers of the tow are substantially parallel to the major axis of the article and extend from about 1/2 to substantially the length of the article.

13. The article of Claim 11 wherein the fibers of the tow are substantially parallel to the major axis of the diaper and extend substantially the length of the diaper.

14. The article of Claim 11 wherein said absorbant core comprises fluff pulp and superabsorbant powder.

15. The absorbant article of Claim 11 which is a diaper containing up to 3 tows of Claim 10 wherein the major axis of each tow lies between ±30° around the major axis of the diaper and wherein the tows lie either just beneath the top sheet or lie intimately mixed with the absorbant core or lie adjacent to the back sheet.

16. Staple fibers comprising a plurality of cut fibers of Claim 1.

17. An absorbant article comprising a diaper or incontinent pad having a major axis and a minor axis and a length in excess of a width comprising a top sheet, a back sheet, and an absorbant core comprising at least one absorbant layer wherein said core comprises an intimate blend of the staple fiber of Claim 12 with fluff pulp.

18. The fiber of Claim 1 incorporated into one or more of the following: a tampon, a sweat absorbing headband or wristband, a surgical sponge or a wound dressing, a sweat absorbing and distributing insole for footwear, a wipe, a fabric softener strip for use in clothes dryers, a wound drain or surgical drain, a towel, a geotextile, athletic socks, a jogging suit, a cosmetic applicator, a furniture polish applicator, a pap smear sampler, a throat culture sampler, a blood-analyzer test element, an ink cartridge, a household and industrial deodorizer, a humidifier fabric, a moist filter media, an orthopaedic cast liner, or spunbonded structures.

19. A spinneret having at least one orifice substantially as described in Figure 3 wherein W is between 0.064 mm and 0.12 mm;

$X_2$ is $4W \,{}^{+4W}_{-1W}$; $X_4$ is $2W \pm 0.5W$; $X_6$ is $6W \,{}^{+4W}_{-2W}$; $X_8$ is $6W \,{}^{+5W}_{-2W}$; $X_{10}$ is $7W \,{}^{+5W}_{-2W}$; $X_{12}$ is $9W \,{}^{+5W}_{-1W}$; $X_{14}$ is $10W \,{}^{+5W}_{-2W}$; $X_{16}$ is $11W \,{}^{+5W}_{-2W}$; $X_{18}$ is $6W \,{}^{+5W}_{-2W}$; $\theta_2$ is 30° ± 30°; $\theta_4$ is 45° ± 45°; $\theta_6$ is 30° ± 30°; and $\theta_8$ is 45° ± 45°.

20. A spinneret having at least one orifice substantially as described in Figure 4 wherein W is between 0.64 mm and 0.12 mm; $X_{20}$ is $17W \,{}^{+5W}_{-2W}$; $X_{22}$ is $3W \pm W$; $X_{24}$ is $4W \pm 2W$; $X_{26}$ is $60W \,{}^{+8W}_{-4W}$; $X_{28}$ is $17W \,{}^{+5W}_{-2W}$; $X_{30}$ is $2W \pm 0.5W$; $X_{32}$ is $72W \,{}^{+10W}_{-5W}$; and $\theta_{10}$ is 45° ± 15°.

21. A spinneret having at least one orifice substantially as described in Figure 5 wherein W is between 0.064 mm and 0.12 mm; $X_{34}$ is $2W \pm 0.5W$; $X_{36}$ is $58W \,{}^{+20W}_{-10W}$; $X_{38}$ is $24W \,{}^{+20W}_{-6W}$; $\theta_{12}$ is $20° \,{}^{+15°}_{-10°}$; $\theta_{14}$ is $\dfrac{180° - 2\theta_{12}}{n-1}$; and n = number of legs per 180° = 2 to 6.

22. A spinneret having at least one orifice substantially as described in Figure 6 wherein W is between 0.064 mm and 0.12 mm; $X_{42}$ is $6W \,{}^{+4W}_{-2W}$; $X_{44}$ is 11W ± 5W; $X_{46}$ is 11W ± 5W; $X_{48}$ is 24W ± 10W; $X_{50}$ is 38W ± 13W; $X_{52}$ is $3W \,{}^{+3W}_{-1W}$; $X_{54}$ is $6W \,{}^{+6W}_{-2W}$; $X_{56}$ is 11W ± 5W; $X_{58}$ is 7W ± 5W; $X_{60}$ is 17W ± 7W; $X_{62}$ is 28W ± 11W; $X_{64}$ is 24W ± 10W; $X_{66}$ is 17W ± 7W; $X_{68}$ is 2W ± 0.5W; $\theta_{16}$ is $45° \,{}^{+30°}_{-15°}$; $\theta_{18}$ is 45° ± 15° and $\theta_{20}$ is 45° ± 15°.

23. A spinneret having at least one orifice substantially as described in Figure 7 wherein W is between 0.064 mm and 0.12 mm; $X_2$ is $4W \,{}^{+4W}_{-1W}$;

$^{+4W}_{-1W}$; $X_4$ is $2W \pm 0.5W$; $X_6$ is $6W\ ^{+4W}_{-2W}$; $X_8$ is $6W\ ^{+5W}_{-2W}$; $X_{10}$ is $7W\ ^{+5W}_{-2W}$; $X_{12}$ is $9W\ ^{+5W}_{-1W}$; $X_{14}$ is $10W\ ^{+5W}_{-2W}$; $X_{16}$ is $11W\ ^{+5W}_{-2W}$; $X_{18}$ is $6W\ ^{+5W}_{-2W}$; $\theta$

$\theta_2$ is 30° ± 30°; $\theta_4$ is 45° ± 45°; $\theta_5$ is 30° ± 30°; and $\theta_8$ is 45° ± 45°.

24. A spinneret having at least one orifice substantially as described in Figure 8 wherein W is between 0.064 mm and 0.12 mm;

$X_2$ is $4W\ ^{+4W}_{-1W}$; $X_4$ is $2W \pm 0.5W$; $X_6$ is $6W\ ^{+4W}_{-2W}$; $X_8$ is $6W\ ^{+5W}_{-2W}$; $X_{10}$ is $7W\ ^{+5W}_{-2W}$; $X_{12}$ is $9W\ ^{+5W}_{-1W}$; $X_{14}$ is $10W\ ^{+5W}_{-2W}$; $X_{16}$ is $11W\ ^{+5W}_{-2W}$; $X_{18}$ is $6W\ ^{+5W}_{-2W}$;

$\theta_2$ is 30° ± 30°; $\theta_4$ is 45° ± 45°; $\theta_5$ is 30° ± 30°; and $\theta_8$ is 45° ± 45°.

25. A spinneret having at least one orifice substantially as described in Figure 6A wherein W is between 0.064 mm and 0.12 mm,

$X_{72}$ is $8W\ ^{+4W}_{-2W}$, $X_{74}$ is $8W\ ^{+4W}_{-2W}$,

$X_{76}$ is 12W ± 4W, $X_{78}$ is 8W ± 4W, $X_{80}$ is 24W ± 12W, $X_{82}$ is 18W ± 6W, $X_{84}$ is $8W\ ^{+4W}_{-2W}$,

$X_{86}$ is 16W ± 6W, $X_{88}$ is 24W ± 12W, $X_{90}$ is 18W ± 6W, $X_{92}$ is 2W ± 0.5W, $\theta_{22}$ is 135° ± 30°, $\theta_{24}$ is 90° $\pm\ ^{45°}_{30°}$,

$\theta_{26}$ is 45° ± 15°, $\theta_{28}$ is 45° ± 15°, $\theta_{30}$ is 45° ± 15°, $\theta_{32}$ is 45° ± 15°, $\theta_{34}$ is 45° ± 15°, $\theta_{36}$ is 45° ± 15°, and $\theta_{38}$ is 45° ± 15°.

26. A process for preparing a fiber that satisfies the equation
$(1\text{-}X \cos \theta_a) < 0$,
wherein $\theta_a$ is the advancing contact angle of water measured on a flat film made from the same material as the fiber and having the same surface treatment,
X is a shape factor of the fiber cross-section that satisfies the following equation

$$(1 - X \cos \theta_a) < 0,$$

wherein
$P_w$ is the wetted perimeter of the fiber and r is the radius of the circumscribed circle circumscribing the fiber cross-section and D is the minor axis dimension across the fiber cross-section,
comprising heating a fiber-forming material at or above its melting point and extruding said heated material through a spinneret having at least one orifice capable of forming the desired fiber, and applying an appropriate surface treatment.

27. A process for spontaneously transporting an aqueous fluid comprising contacting a synthetic fiber which is capable of spontaneously transporting water on the surface thereof wherein said fiber satisfies the equation
$(1\text{-}X \cos \theta_a) < 0$,
wherein
$\theta_a$ is the advancing contact angle of water measured on a flat film made from the same material as the fiber and having the same surface treatment, if any,
X is a shape factor of the fiber cross-section that satisfies the following equation

$$X = \frac{P_w}{4r + (\pi-2)D}$$

wherein
$P_w$ is the wetted perimeter of the fiber and r is the radius of the circumscribed circle circumscribing the fiber cross-section and D is the minor axis dimension across the fiber cross-section,
with an aqueous fluid.

28. A process for spontaneously transporting an alcoholic fluid comprising contacting a synthetic fiber which is capable of spontaneously transporting water on the surface thereof wherein said fiber satisfies the equation
$(1\text{-}X \cos \theta_a) < 0$,
wherein
$\theta_a$ is the advancing contact angle of water measured on a flat film made from the same material as the fiber and having the same surface treatment, if any,
X is a shape factor of the fiber cross-section that satisfies the following equation

$$X = \frac{P_w}{4r + (\pi-2)D}$$

wherein
$P_w$ is the wetted perimeter of the fiber and r is the radius of the circumscribed circle circumscribing the fiber cross-section and D is the minor axis dimension across the fiber cross-section,
with an alcoholic fluid.

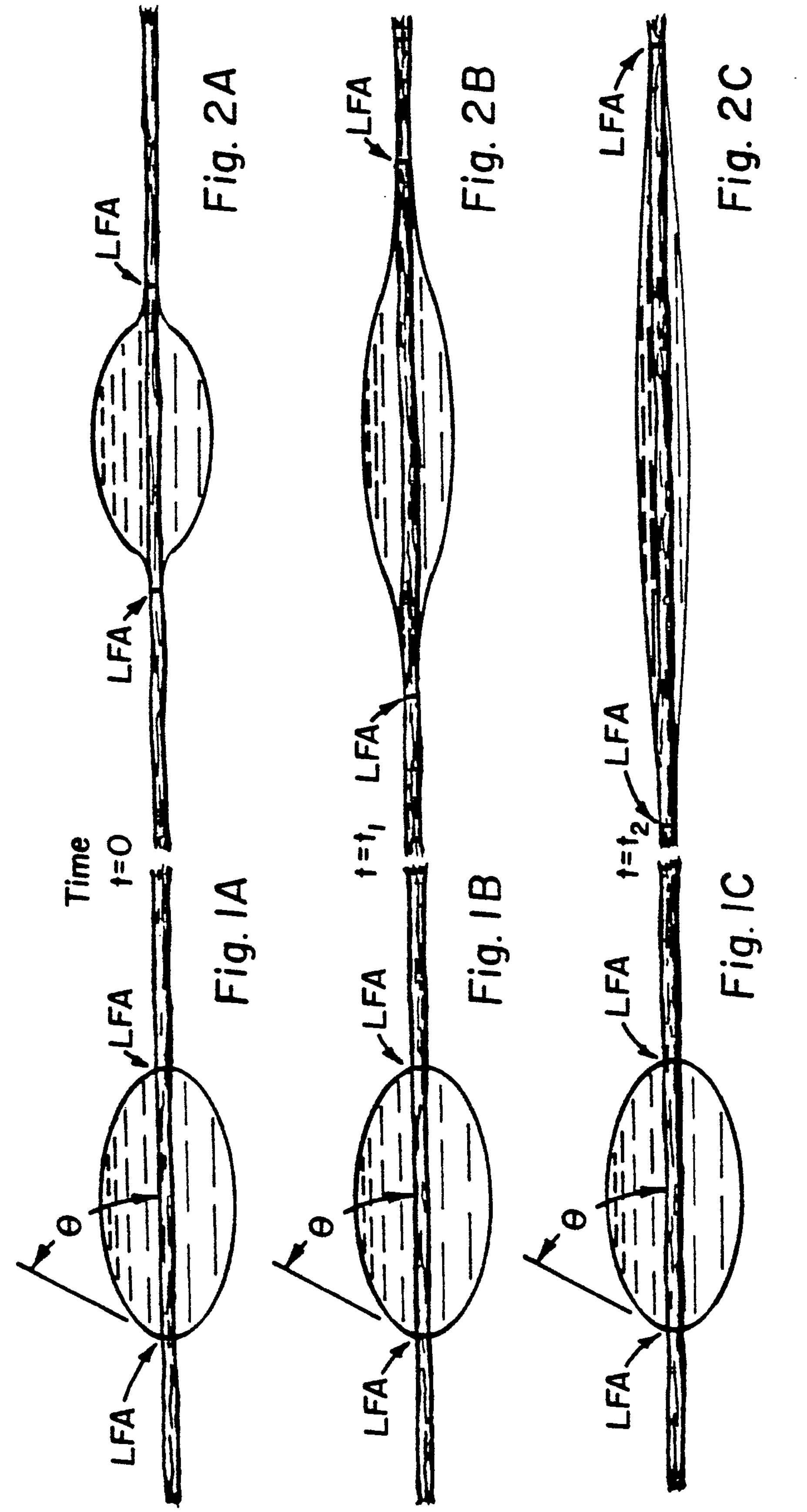


Fig. 1A

Fig. 1B

Fig. 1C

Fig. 2A

Fig. 2B

Fig. 2C

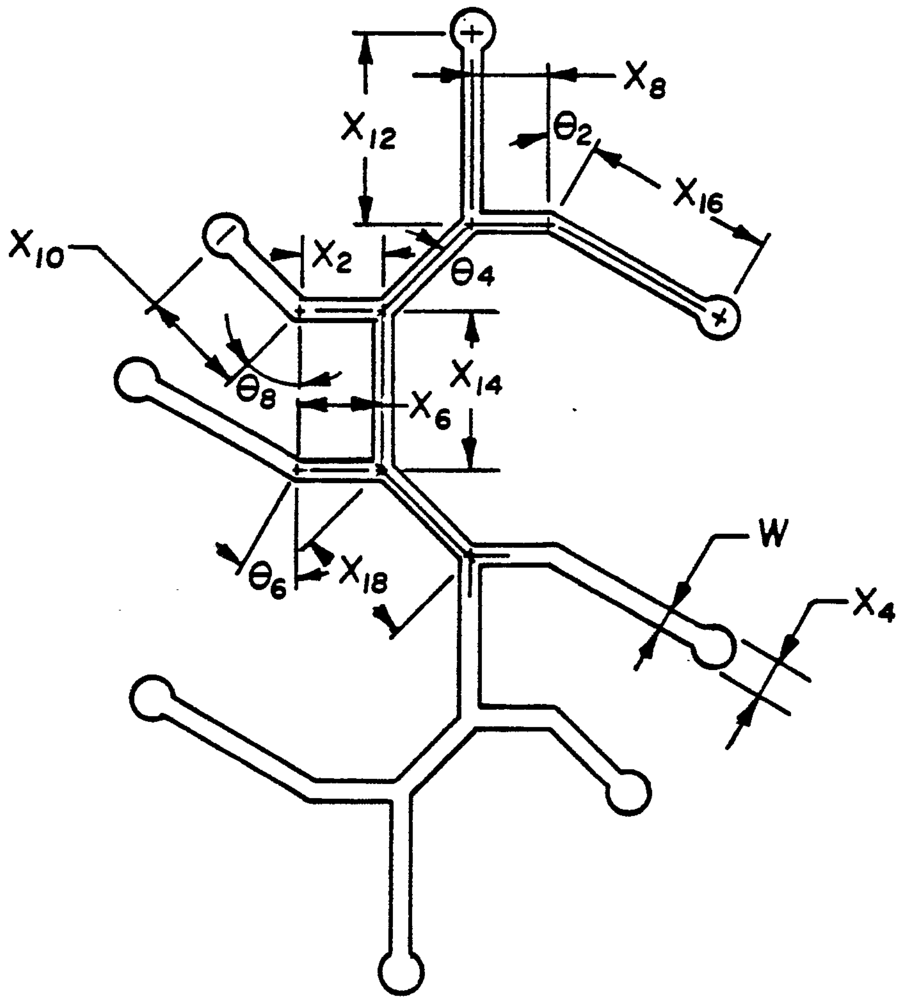


Fig. 3

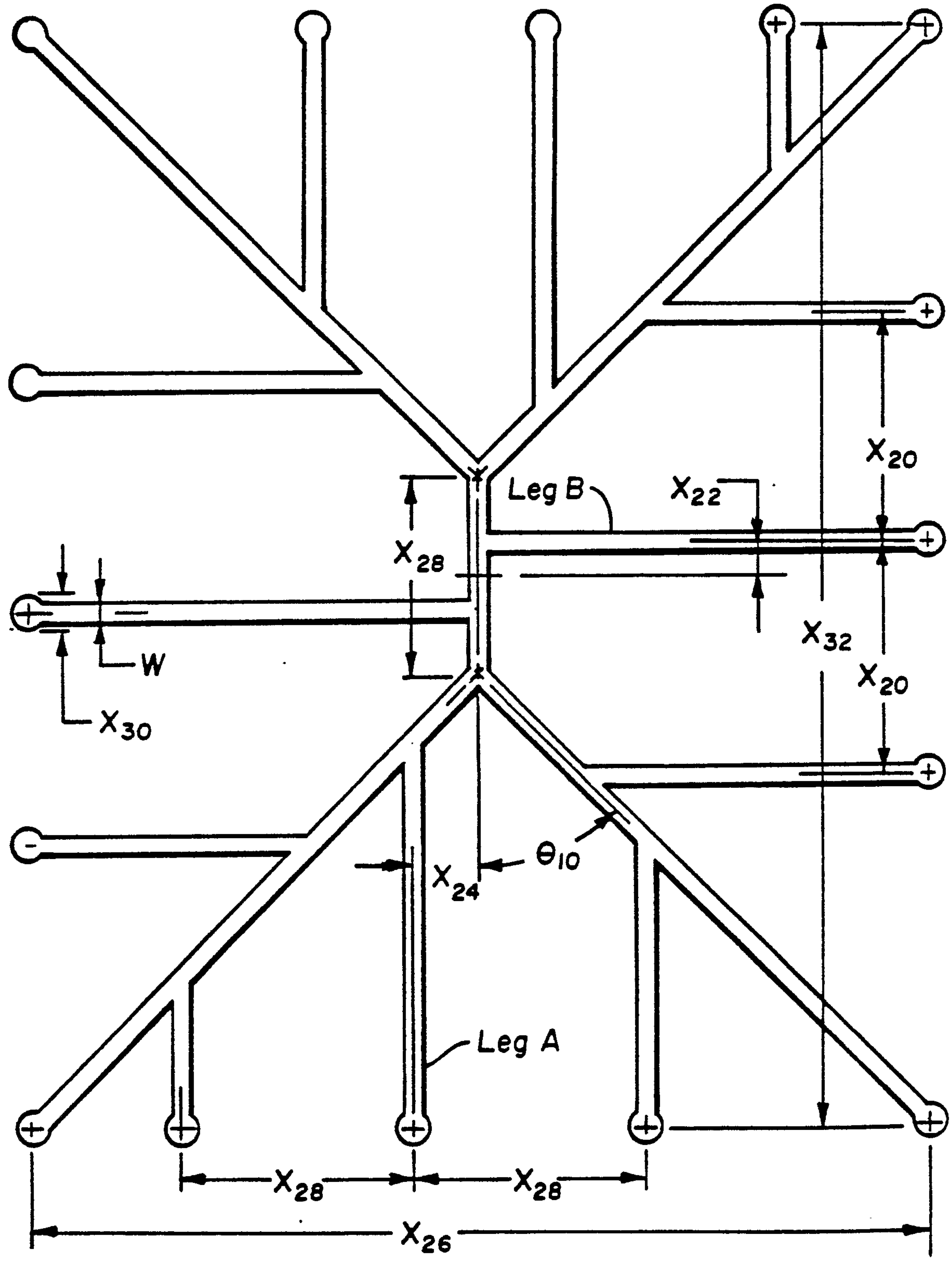


Fig. 4

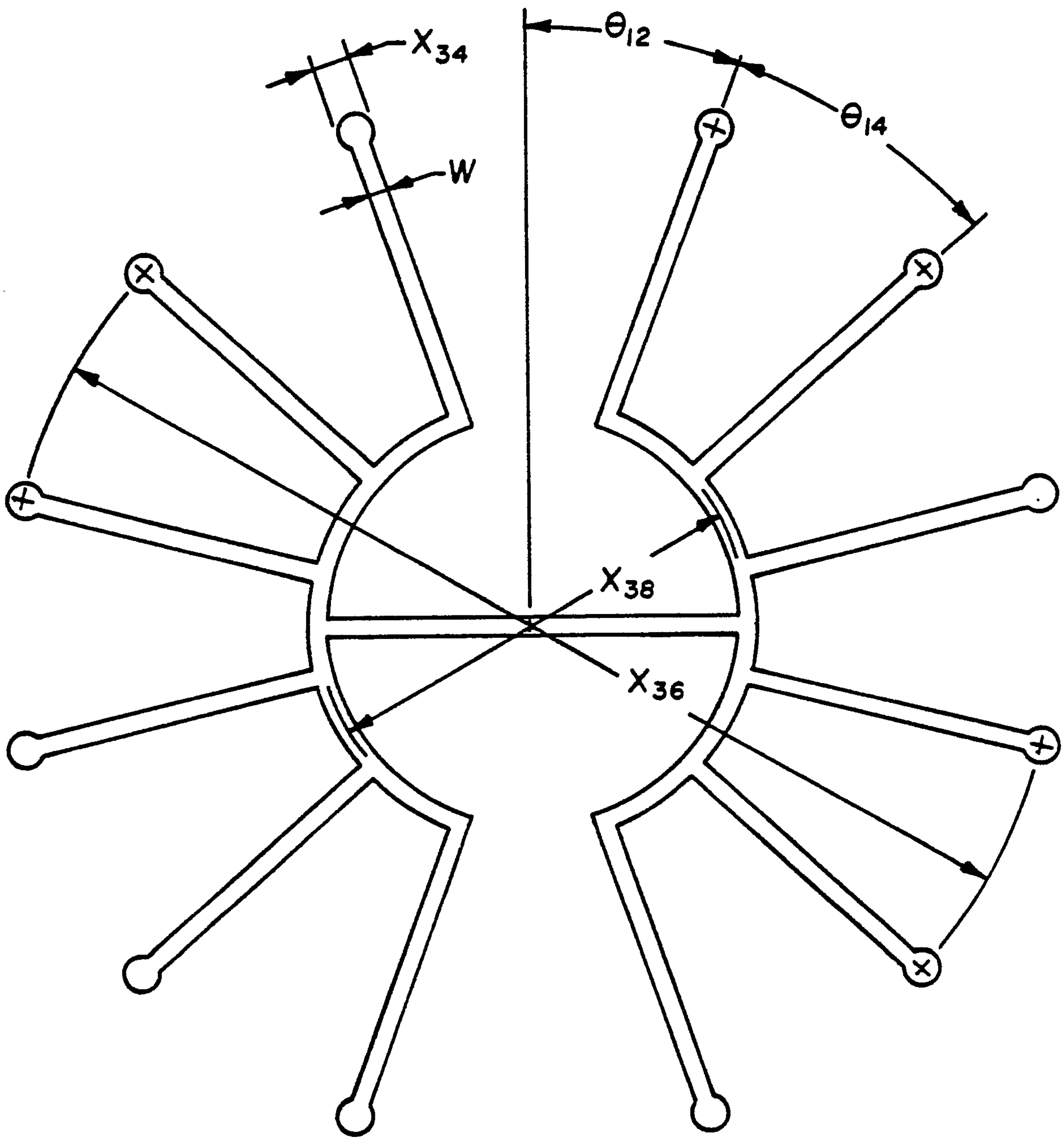


Fig. 5

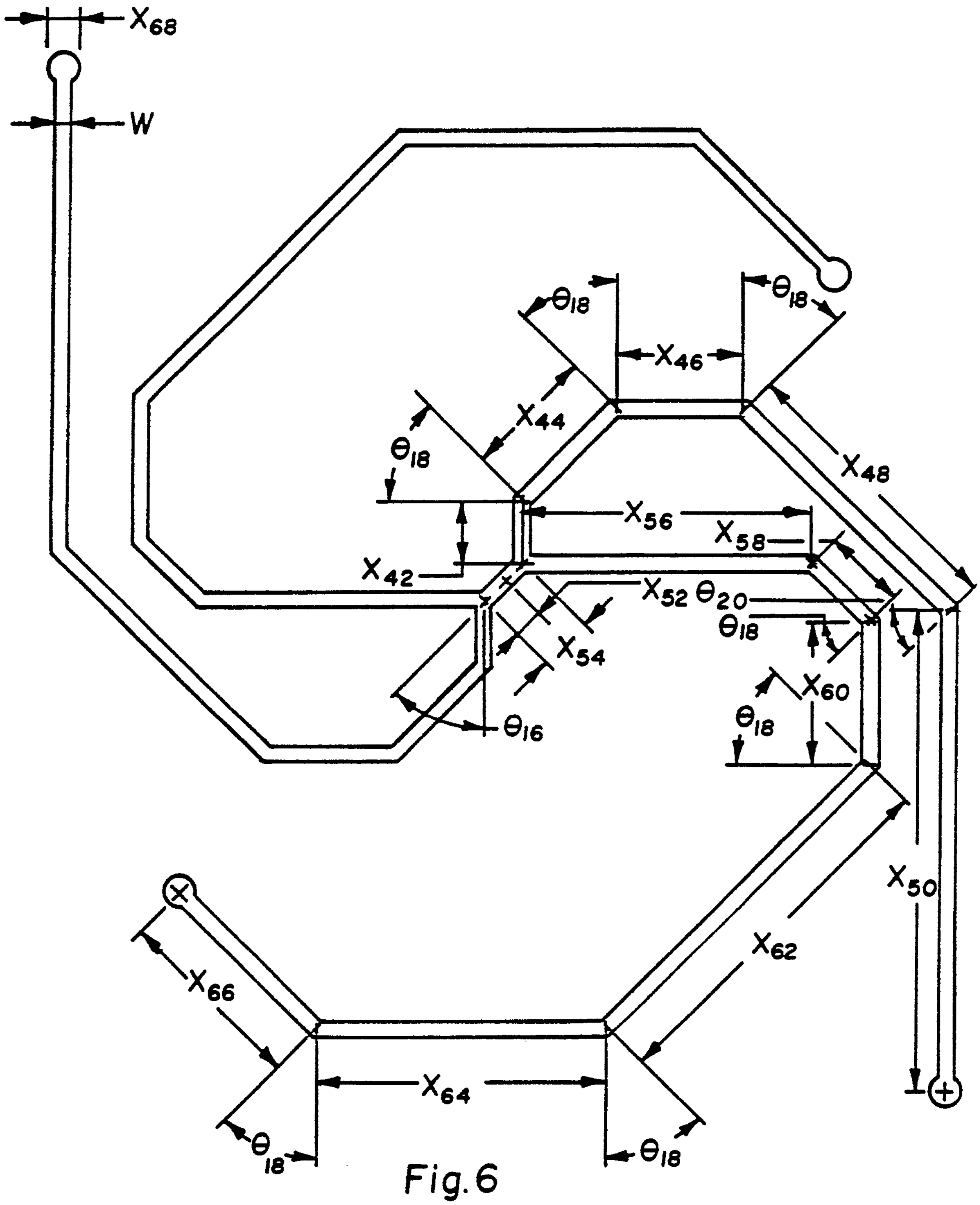
X68
W
θ18
θ18
X46
X44
θ18
X48
X56
X58
X42
X52 θ20
θ18
X54
X60
θ18
θ16
X50
X62
X66
X64
θ18
θ18

Fig. 6

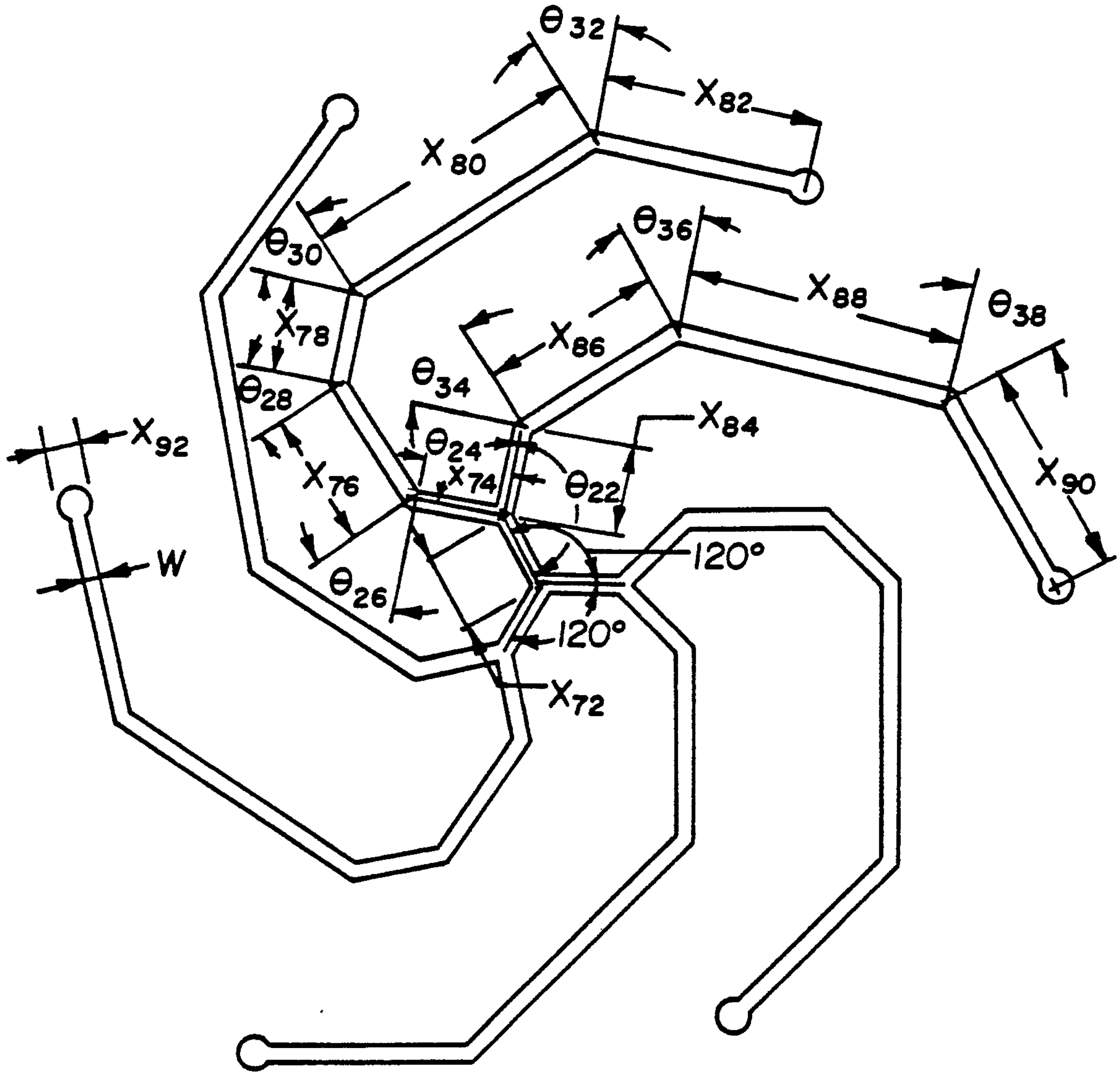
θ32
X82
X80
θ30
θ36
X78
X88
θ38
X86
θ28
θ34
X84
X92
θ24
X74
X76
θ22
X90
120°
W
θ26
120°
X72

Fig. 6 B

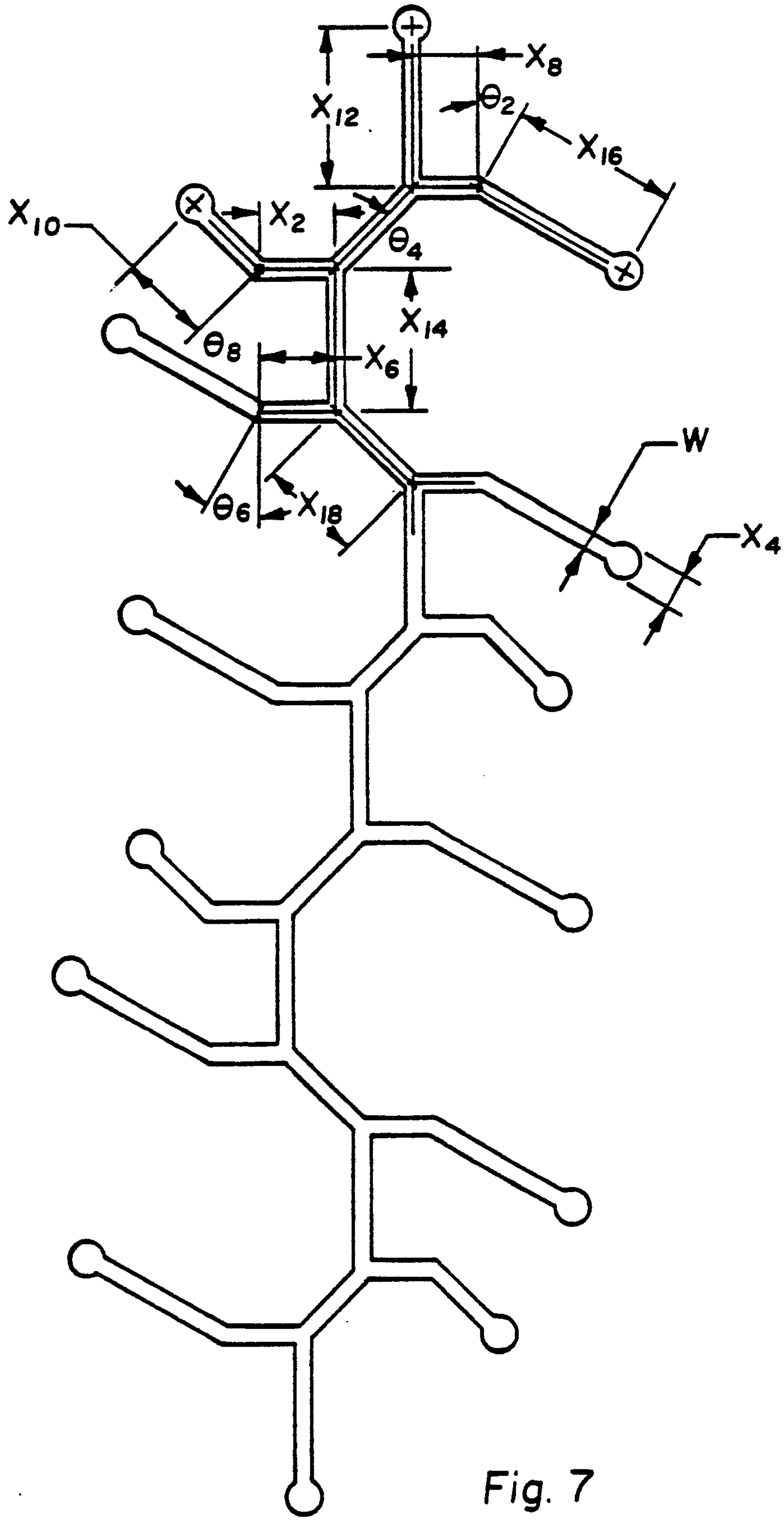


Fig. 7

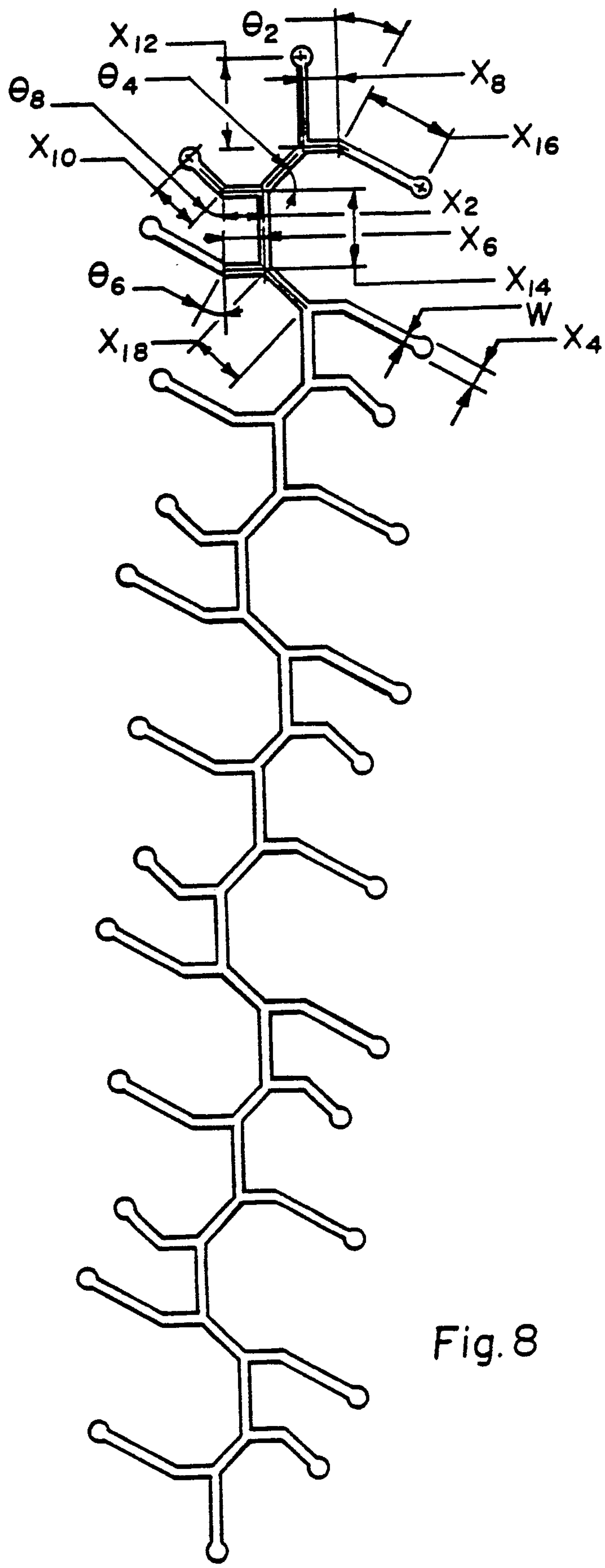


Fig. 8

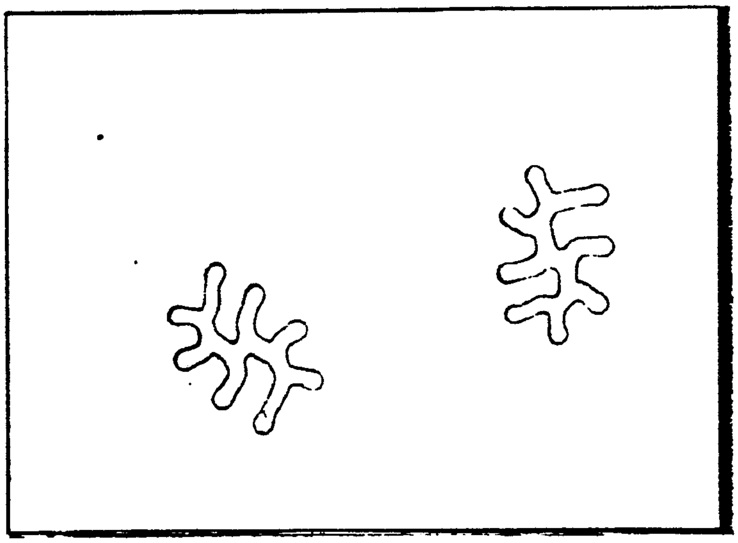

Fig. 9

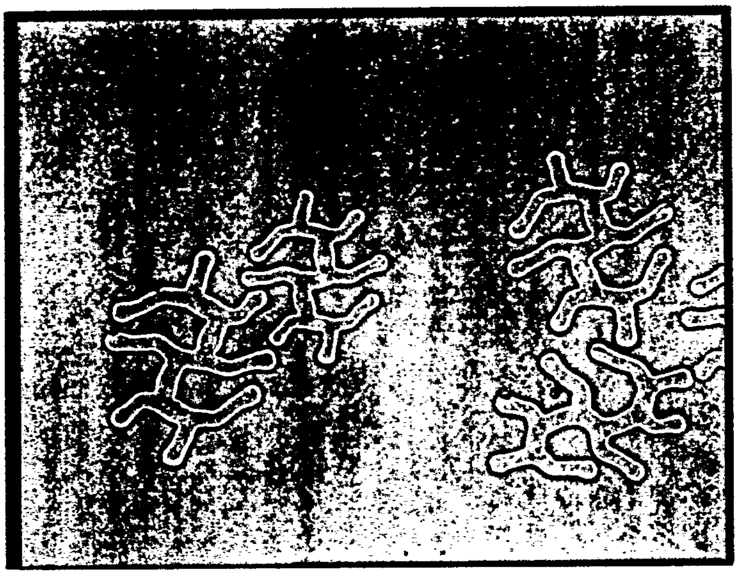

Fig. 10

Nouvellement déposé

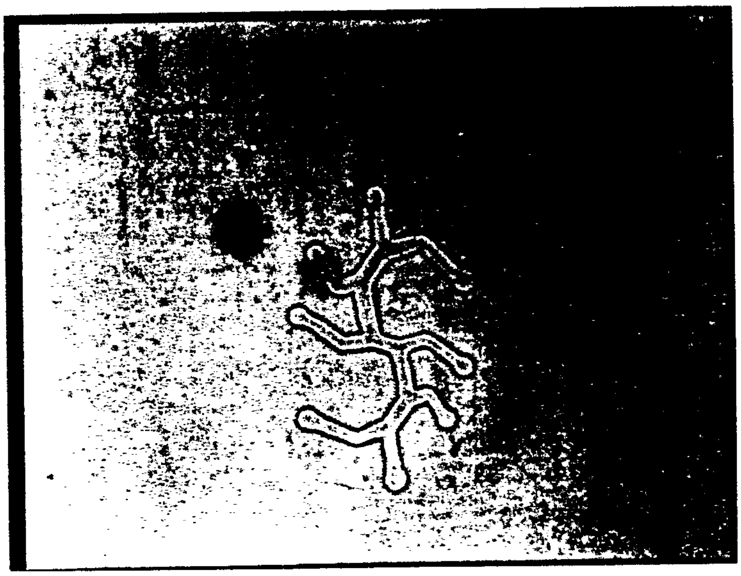

*Fig. 11*

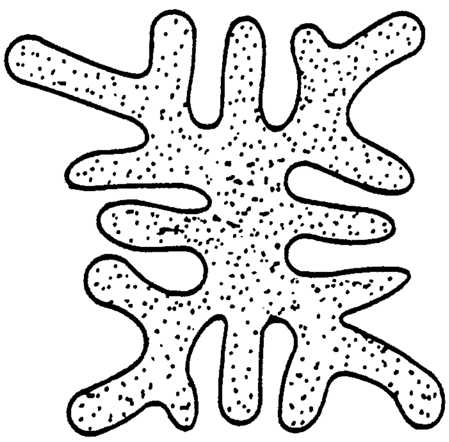

Fig. 12

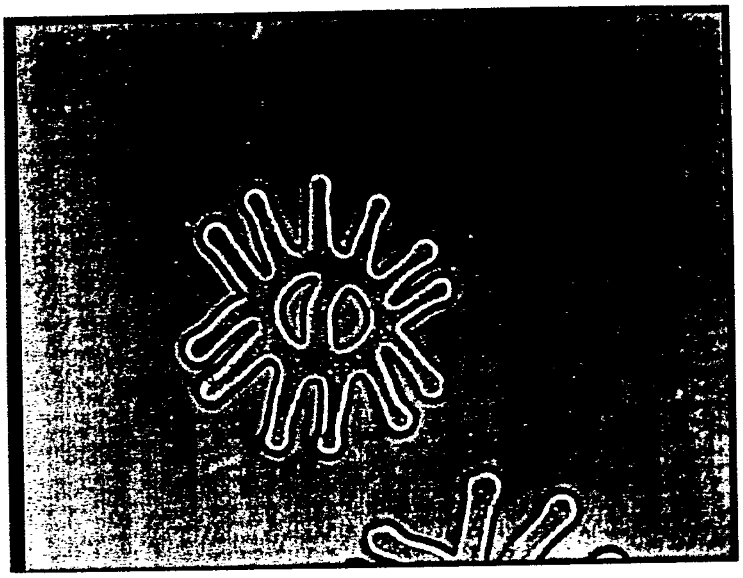

Fig. 13

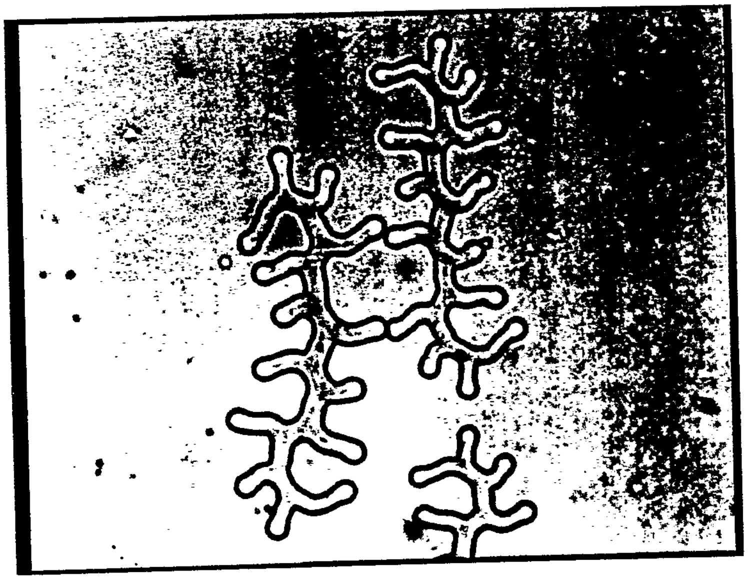

Fig. 14

Nouvellement déposé

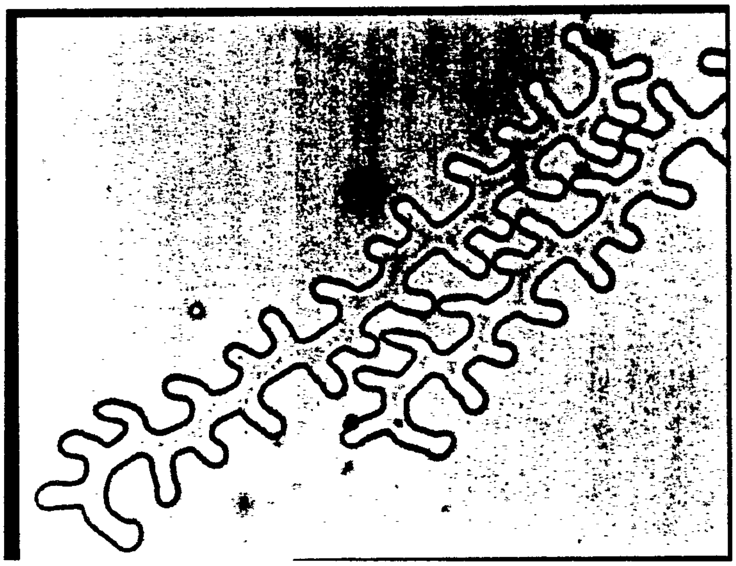

Fig. 15

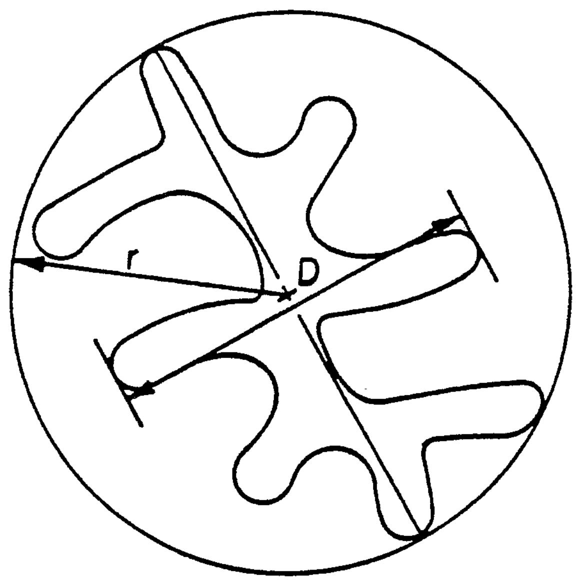


Fig. 16

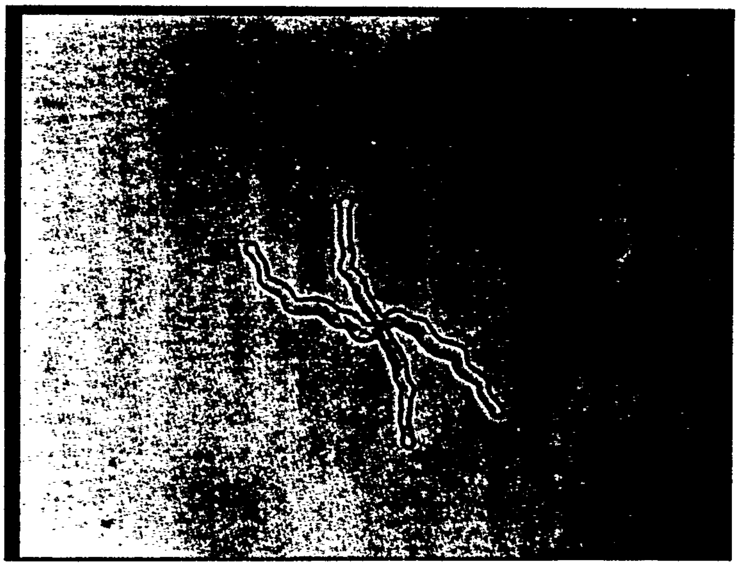

Fig. 17

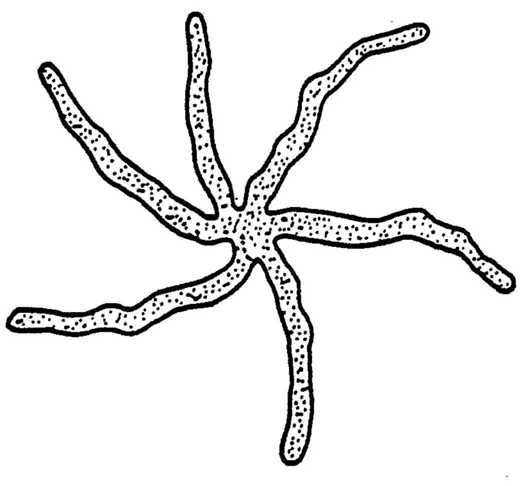

Fig. 17 B

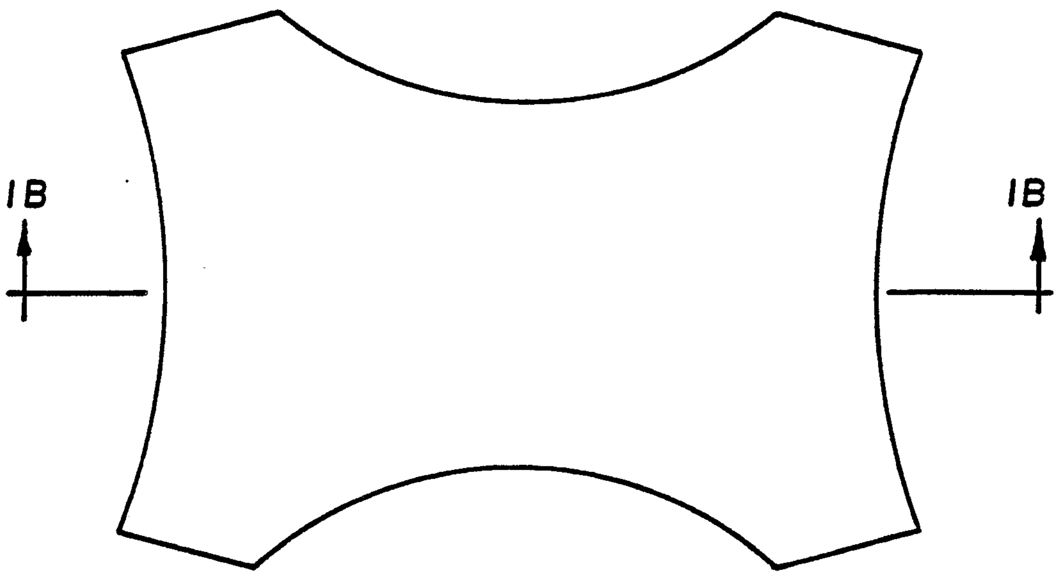


Fig. 18 A

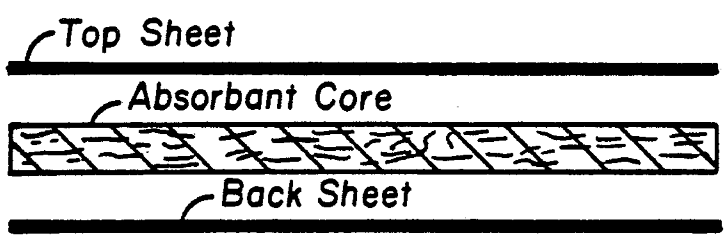


Fig. 18 B

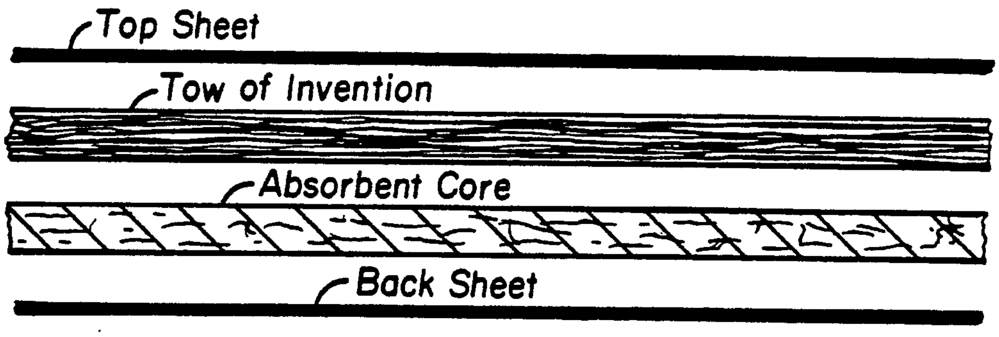
Top Sheet
Tow of Invention
Absorbent Core
Back Sheet

Fig. 19

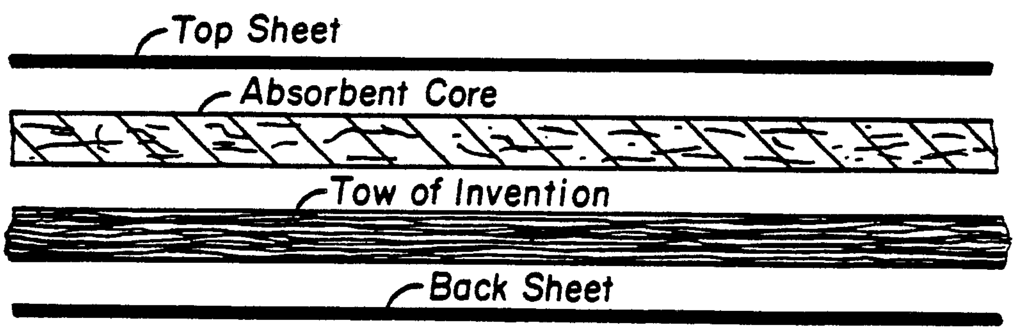
Top Sheet
Absorbent Core
Tow of Invention
Back Sheet

Fig. 20

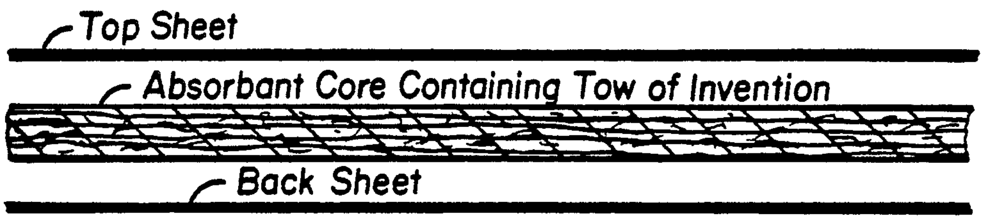
Top Sheet
Absorbant Core Containing Tow of Invention
Back Sheet

Fig. 21

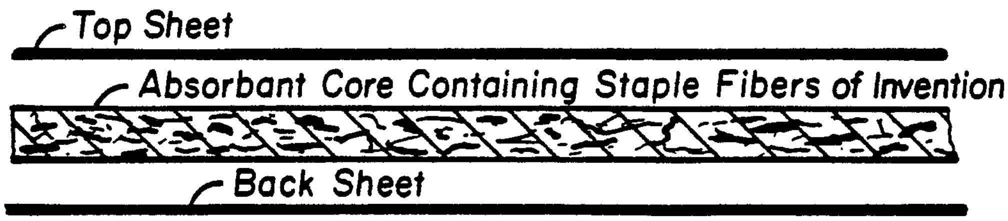
Top Sheet
Absorbant Core Containing Staple Fibers of Invention
Back Sheet

Fig. 22

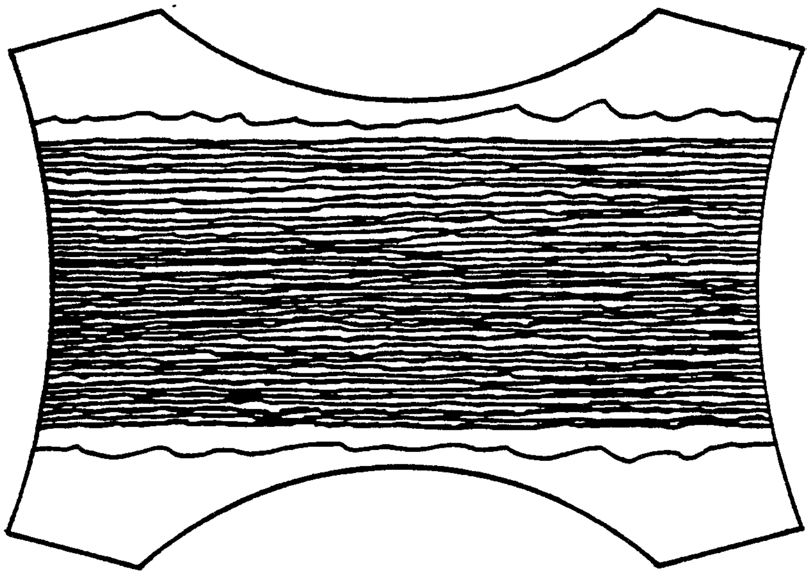

Fig. 23 A

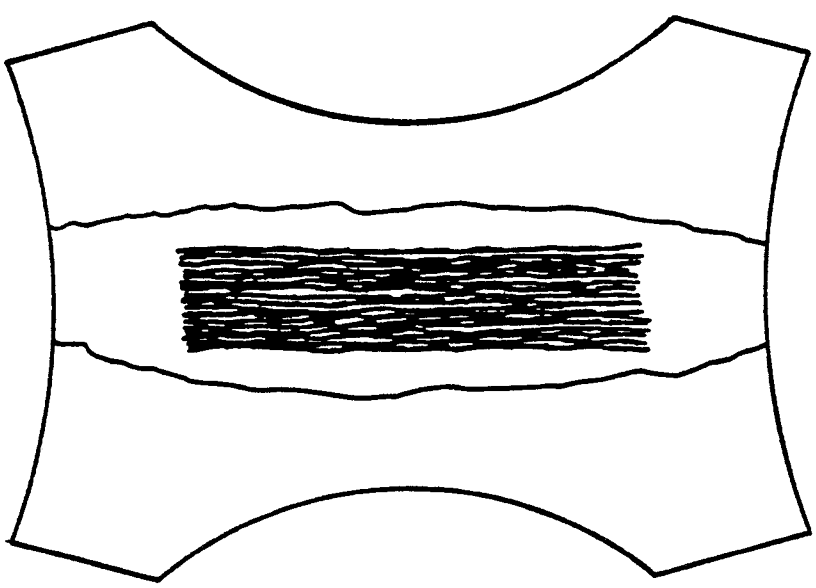

Fig. 23 B

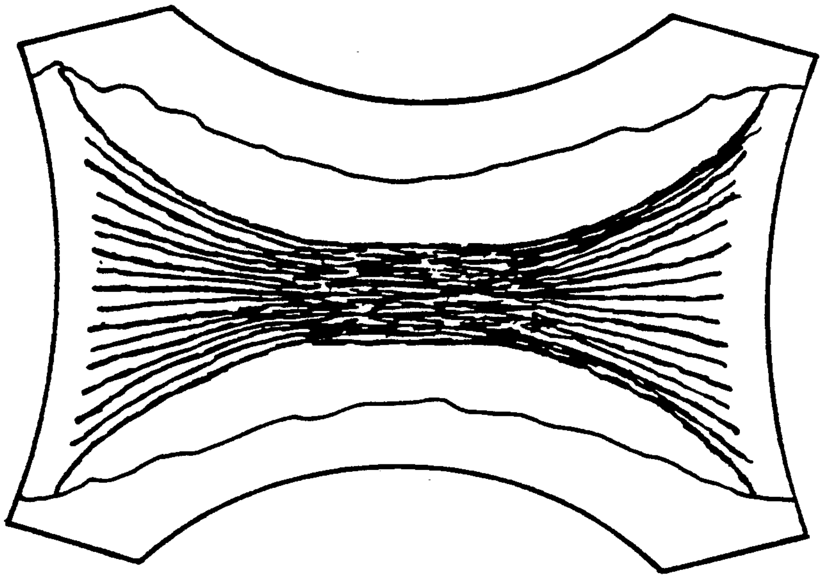

Fig. 24

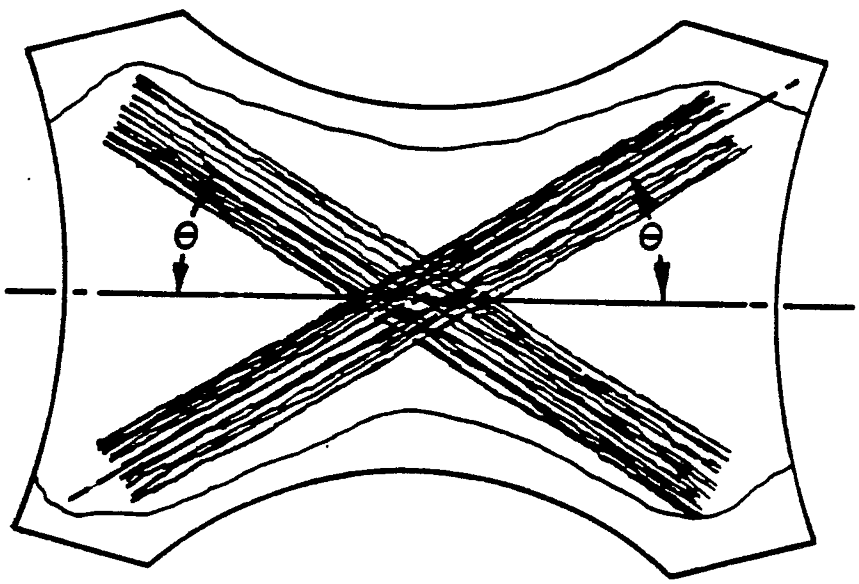


Fig. 25

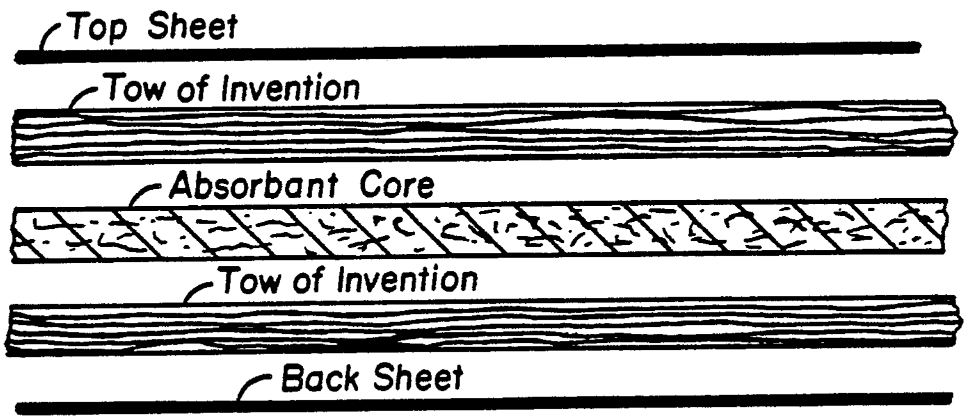
Top Sheet
Tow of Invention
Absorbant Core
Tow of Invention
Back Sheet

Fig. 26

Fig. 27

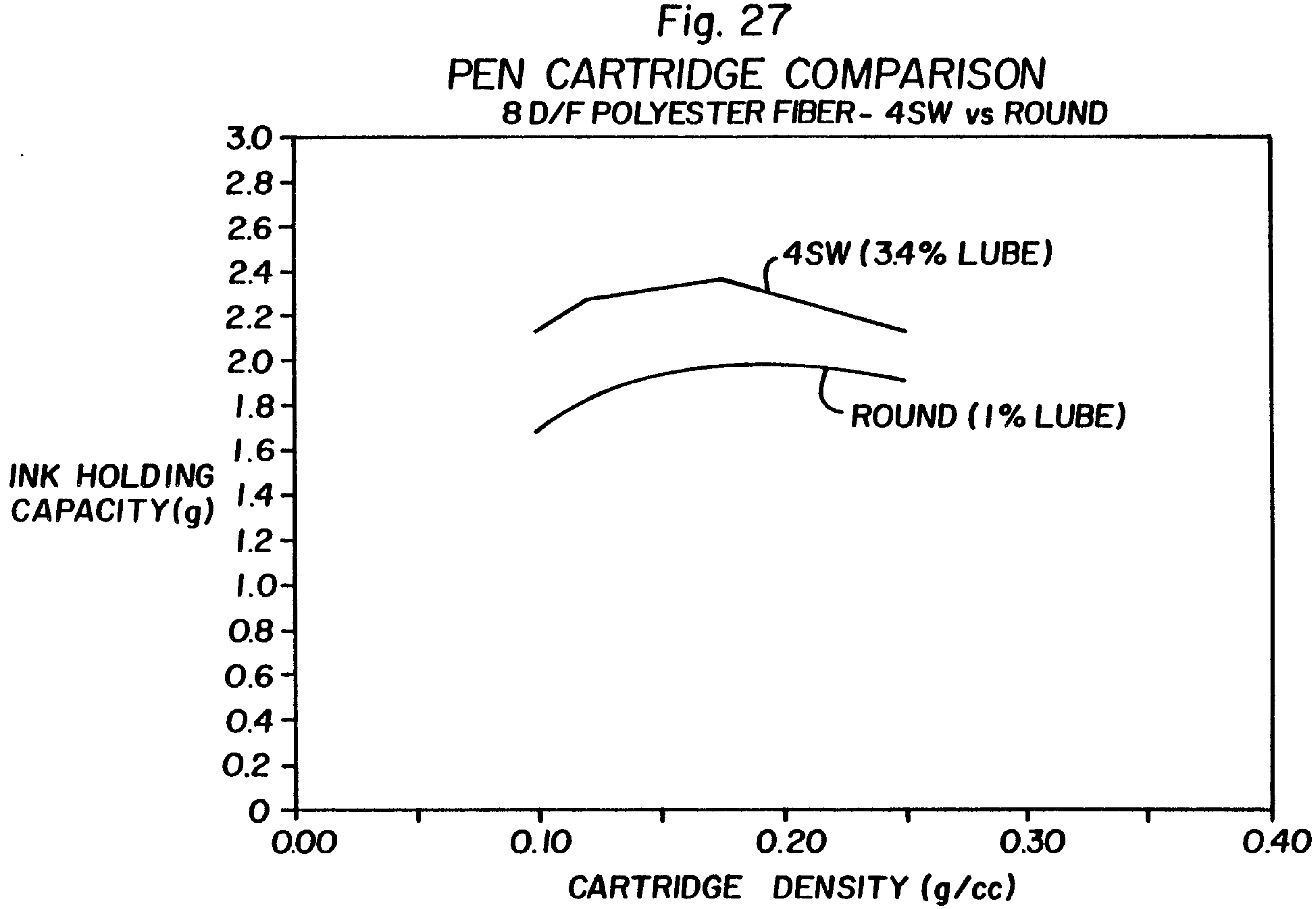
PEN CARTRIDGE COMPARISON
8 D/F POLYESTER FIBER - 4SW vs ROUND
4SW (3.4% LUBE)
ROUND (1% LUBE)
INK HOLDING CAPACITY (g)
3.0
2.8
2.6
2.4
2.2
2.0
1.8
1.6
1.4
1.2
1.0
0.8
0.6
0.4
0.2
0
0.00
0.10
0.20
0.30
0.40
CARTRIDGE DENSITY (g/cc)

Fig. 28

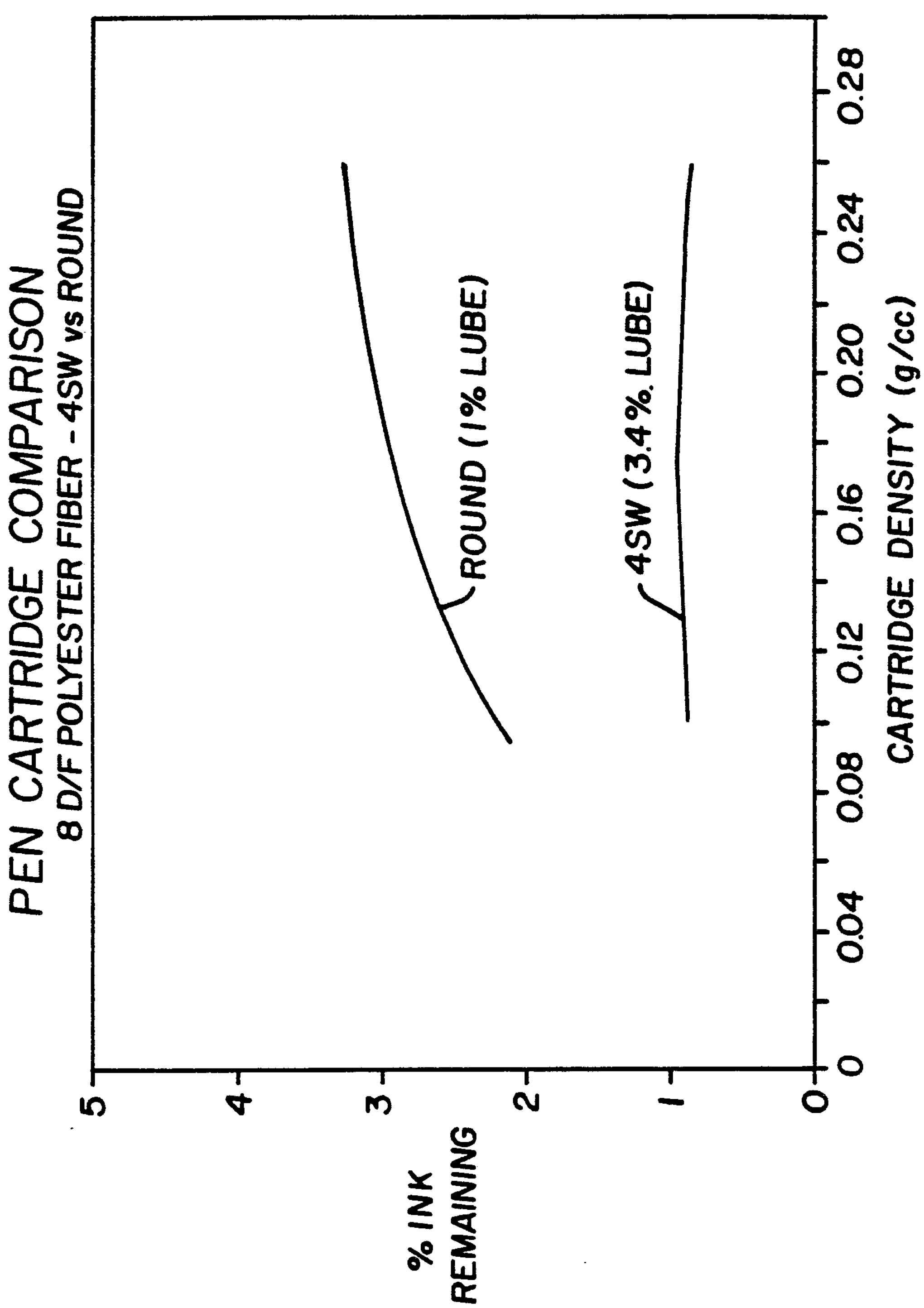
PEN CARTRIDGE COMPARISON
8 D/F POLYESTER FIBER - 4SW vs ROUND
ROUND (1% LUBE)
4SW (3.4% LUBE)
% INK REMAINING
0
1
2
3
4
5
CARTRIDGE DENSITY (g/cc)
0
0.04
0.08
0.12
0.16
0.20
0.24
0.28

Fig. 29

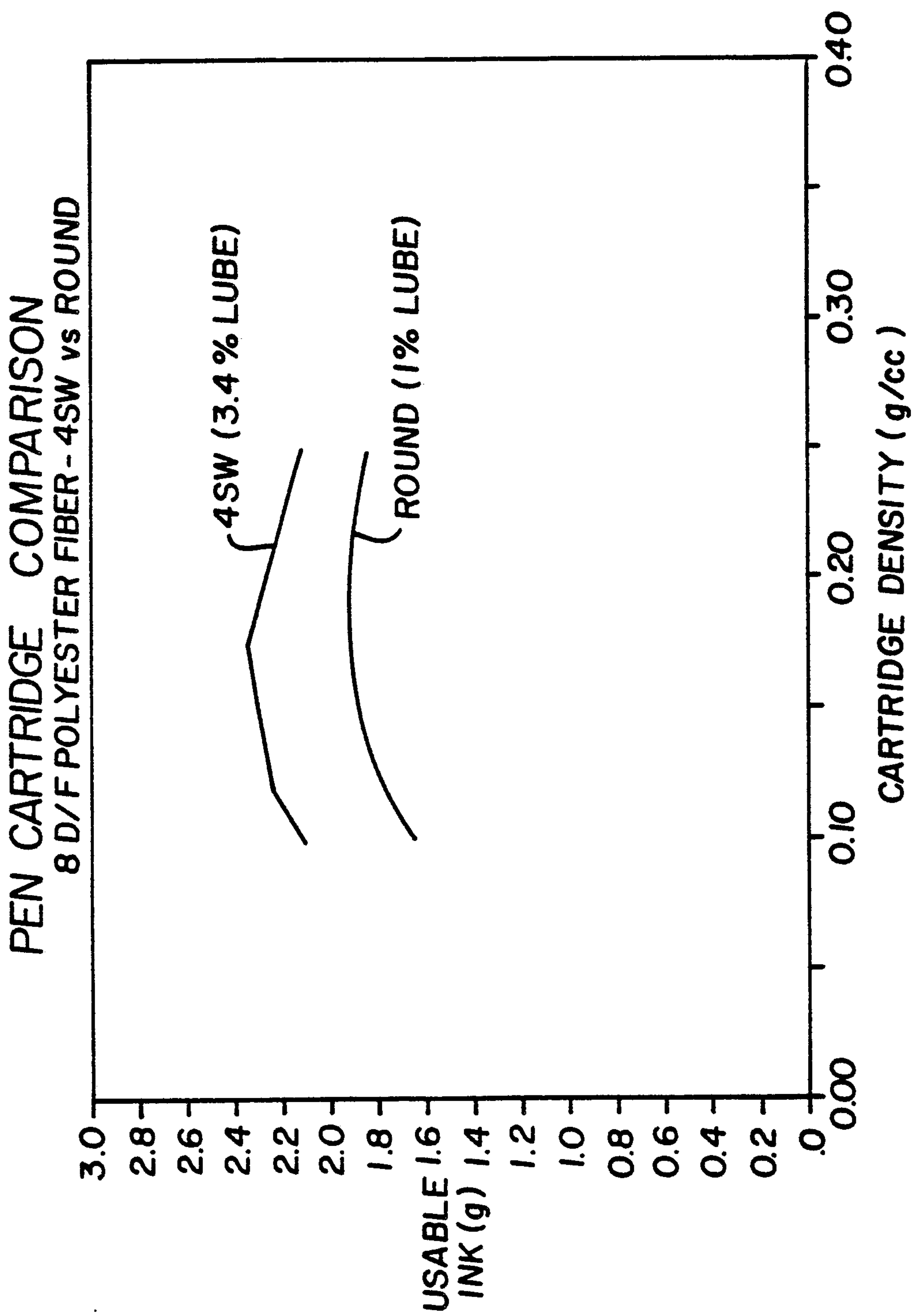
PEN CARTRIDGE COMPARISON
8 D/F POLYESTER FIBER - 4SW vs ROUND
4SW (3.4 % LUBE)
ROUND (1% LUBE)
USABLE INK (g)
3.0
2.8
2.6
2.4
2.2
2.0
1.8
1.6
1.4
1.2
1.0
0.8
0.6
0.4
0.2
0
0.00
0.10
0.20
0.30
0.40
CARTRIDGE DENSITY (g/cc)

Fig 30

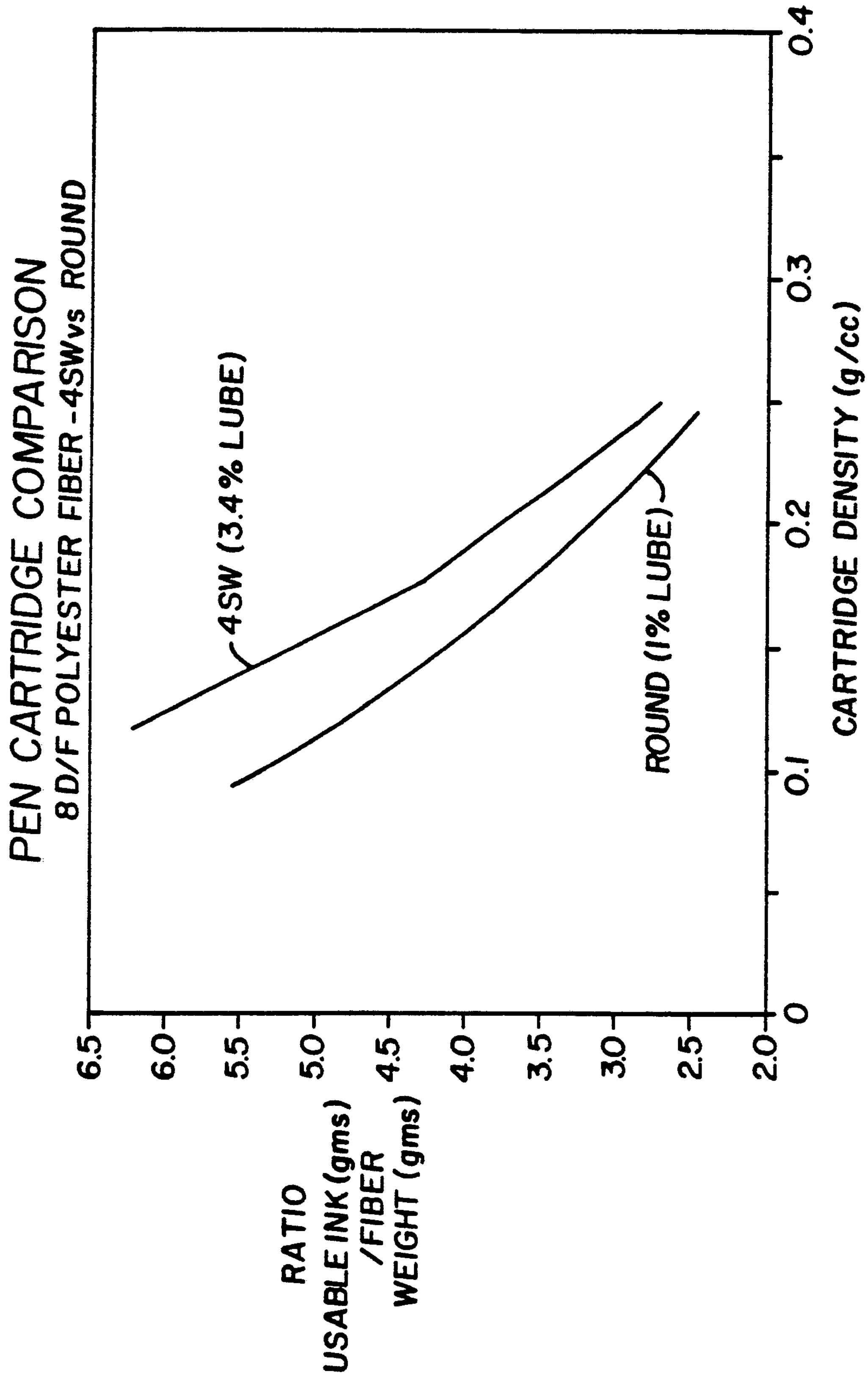
PEN CARTRIDGE COMPARISON
8 D/F POLYESTER FIBER - 4SW vs ROUND
4SW (3.4% LUBE)
ROUND (1% LUBE)
RATIO
USABLE INK (gms)
/FIBER
WEIGHT (gms)
6.5
6.0
5.5
5.0
4.5
4.0
3.5
3.0
2.5
2.0
0
0.1
0.2
0.3
0.4
CARTRIDGE DENSITY (g/cc)

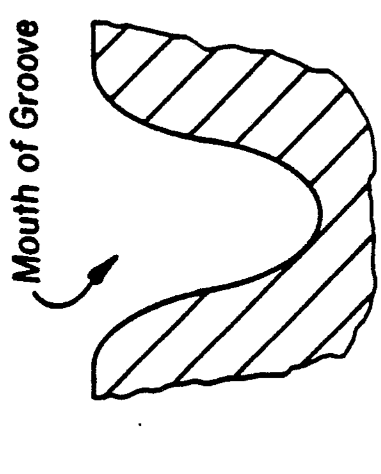

Fig. 31 A

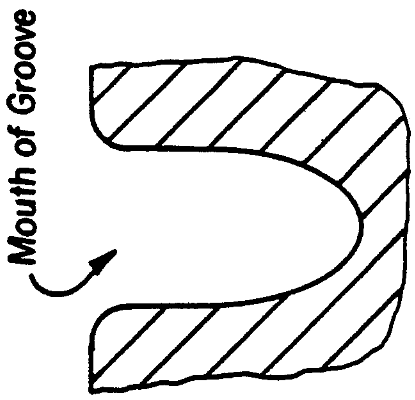

Fig. 31 B

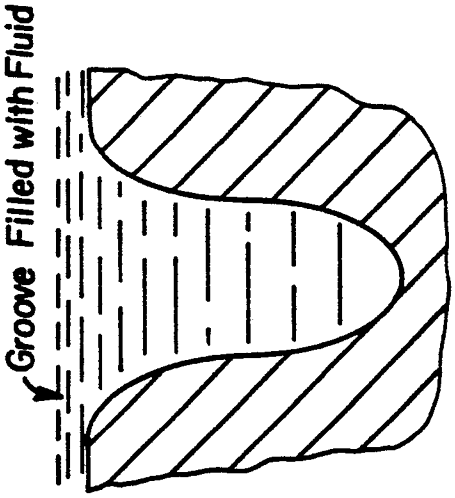

Fig. 31 C

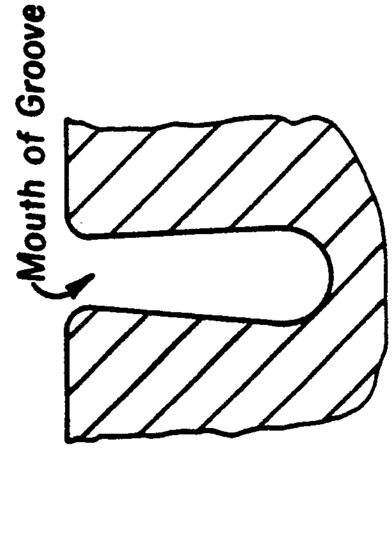

Fig. 32 A

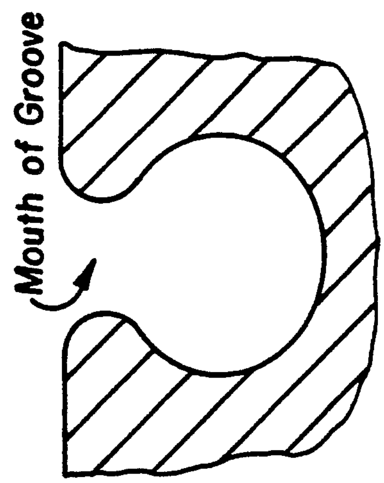

Fig. 32 B

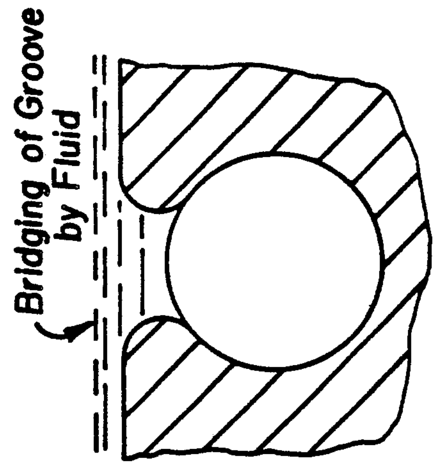

Fig. 32 C